# EUROPEAN PATENT APPLICATION

(11) **EP 4 796 538 A1**
(43) Date of publication of application: **26.08.2026**
(21) Application number: 24880124.3
(22) Date of filing: 17.10.2024
(51) Int. Cl.: C07D 403/14, C07D 401/14, C07D 405/14, C07D 401/04, C07D 241/42, C07D 403/12, C07D 405/12

(54) **NOVEL QUINOXALINE COMPOUNDS, MANUFACTURING METHOD THEREOF, AND USES THEREOF**

(30) Priority: 17.10.2023 KR 20230138635; 16.10.2024 KR 20240141671
(71) Applicant: Gachon University of Industry-Academic Cooperation Foundation, Seongnam-si, Gyeonggi-do 13120 (KR); Gil Medical Center, Incheon 21565 (KR)
(72) Inventor: KIM, Mi-hyun, Incheon 21936 (KR); POGAKU, Vinay, Incheon 21936 (KR); KUMAR, Surendra, Incheon 21936 (KR)
(74) Representative: Abel & Imray LLP
(86) International application number: PCT/KR2024/015716
(87) International publication number: WO 2025/084787

(57) **Abstract**

The present invention relates to novel quinoxaline compounds, methods for their preparation, and pharmaceutical compositions comprising the same as an active ingredient for the prevention or treatment of cancer. The compounds according to the present invention exhibit high inhibitory activity against RET and RET mutations, and thus can be usefully used for the treatment of cancers in which RET and RET mutations occur.

## Description

### BACKGROUND OF THE INVENTION

### 1. Field of the Invention

The present invention relates to novel quinoxaline compounds, methods for their preparation, and pharmaceutical compositions containing them as active ingredients for the prevention or treatment of cancer.

### 2. Description of the Related Art

Protein kinases are enzymes that catalyze the phosphorylation of hydroxyl groups located on tyrosine, serine, and threonine residues of proteins, and play a crucial role in growth factor signaling that induces cell growth, differentiation, and proliferation.

To maintain homeostasis, the body's signaling pathways must properly balanced between activation and inactivation. However, mutations or overexpression of specific protein kinases disrupt normal intracellular signaling pathways (primarily by causing signaling to remain continuously active), leading to various diseases such as cancer, inflammation, metabolic disorders, and neurological diseases.

As protein kinase inhibitors, International Patent Applications WO 2016/180536 A1 and WO 2018/087021 A1 have disclosed quinoxaline derivative compounds for the prevention and treatment of cancer.

Meanwhile, RET (Rearranged During Transfection) is a receptor tyrosine kinase that can be activated through chromosomal rearrangements; it is known that abnormalities in the RET gene (point mutations, chromosomal translocations, inversions, and gene amplification) contribute to carcinogenesis. Abnormal RET activation is responsible for the development of numerous malignant tumors, including non-small cell lung cancer (NSCLC), metastatic colorectal cancer (mCRC), differentiated thyroid carcinoma (DTC), papillary thyroid carcinoma (PTC), and medullary thyroid carcinoma (MTC). The prevalence of RET-positive cases was highest in thyroid cancers (MTC, DTC, PTC) at 36.6%, followed by pancreatic cancer(colorectal cancer) (17.3%), breast cancer (6.5%), and lung cancer (over 3%).

Recent clinical oncology has been focusing on biomarkers and biomarker-based therapies. For example, the NCCN Clinical Practice Guidelines in Oncology (NCCN Guidelines) recommend biomarker testing for non-small cell lung cancer (NSCLC), and RET is identified as a key biomarker for personalizing treatment in patients with solid tumors.

Therefore, compounds that inhibit RET kinase and its mutations are highly useful for the prevention and treatment of cancer.

The present invention focuses on the design and synthesis of compounds capable of treating cancers caused by mutations in the RET kinase gene (designated as RET(+)), such as RET(+) thyroid cancer, non-small cell lung cancer, and colorectal cancer.

RET mutations are highly diverse and include point mutations (more than 50 types), gene fusions (more than 60 types), gene deletions, missense mutations in specific codons (e.g., codon 918), amplifications, and rearrangements, all of which are oncogenic factors. Among these mutations, certain point mutations and fusions are particularly common in the aforementioned cancers. Molecular testing, including next-generation sequencing (NGS), has demonstrated that patients with NSCLC and RET fusions exhibit a low tumor mutational burden (TMB) and a high frequency of low programmed cell death protein-1 (PD-L1) expression (referred to as a "cold tumor"), and do not respond well to immunotherapy.

Accordingly, the inventors sought to develop anticancer agents that inhibit RET kinase and its mutations, and completed the present invention by synthesizing novel quinoxaline compounds that exhibit high inhibitory activity against RET kinase and its mutations.

### [Non-patent literature]

(1) Identification of New ALK and RET Gene Fusions from Colorectal and Lung Cancer Biopsies. Nat. Med. 2012, 18 (3), 382-384.
(2) RET Aberrant Cancers and RET Inhibitor Therapies: Current State-of-the-Art and Future Perspectives. Pharmacol. Ther. 2023, 242, 108344.
(3) Precision Therapy for RET-Altered Cancers with RET Inhibitors. Trends Cancer. 2021, 7 (12), 1074-1088.
(4) RET Rearrangements Are Actionable Alterations in Breast Cancer. Nat. Commun. 2018, 9 (1), 4821.
(5) Actionable tumor alterations and treatment protocol enrollment of pediatric and young adult patients with refractory cancers in the National Cancer Institute-Children's Oncology Group Pediatric MATCH trial. Journal of Clinical Oncology, 2022, 40.20: 2224-2234.
(6) Side Chain Investigation of Imidazopyridazine as a Hinge Binder for Targeting Actionable Mutations of RET Kinase. ACS Medicinal Chemistry Letters 2024, 15 (9), 1566.

### SUMMARY OF THE INVENTION

One objective of the present invention is to provide quinoxaline derivatives having a novel structure and exhibiting anticancer activity.

Another objective of the present invention is to provide a method for preparing the quinoxaline derivatives.

A further objective of the present invention is to provide a pharmaceutical composition for the prevention or treatment of cancer containing the aforementioned quinoxaline derivative having anticancer activity as an active ingredient.

To achieve the above objectives,
according to one aspect of the present invention, a compound represented by Chemical Formula I or Chemical Formula IV below, or a pharmaceutically acceptable salt thereof, is provided. 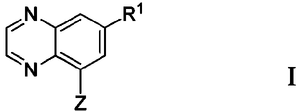 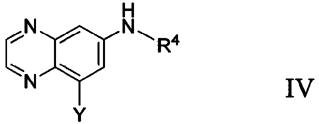 wherein, in chemical formulae I and IV above,
Z is 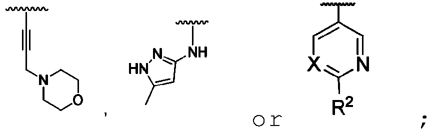
X is CH or N;
R¹is NO₂ or -NR^{a}R^{b},
wherein each of R^{a} and R^{b} is independently H, C₁₋₁₀alkyl, or 5-6 membered heteroarylcarbonyl;
R² is halogen or 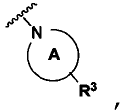
wherein ring A is a 4-6 membered heterocycloalkyl comprising at least one N atom at the point of substitution;
R³ is H, C₁₋₁₀ alkyl, C₁₋₁₀ alkylsulfonyl, C₁₋₃ alkyl substituted with unsubstituted or substituted phenyl, unsubstituted or substituted phenyloxy, unsubstituted or substituted phenyl-CONH-, 5-6 membered heteroaryl, or 6 membered heterocycloalkyl,
wherein the substituted phenyl is each independently substituted with one or more halogens;
Y is halogen; and
R⁴ is C₁₋₁₀ alkyl or 5-6 membered heteroarylcarbonyl.

Furthermore, the present invention provides a method of preparing a compound of Formula IA, comprising reacting a compound of Formula II with a compound of Formula III as shown in Reaction Scheme I below: 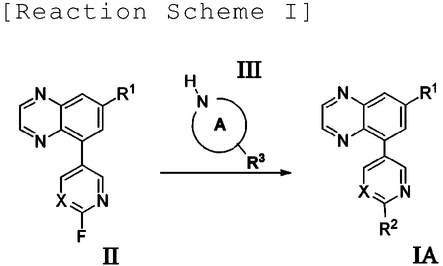 wherein X, R¹, R², ring A, and R³ are as defined in Formula I.

According to another aspect of the present invention, a pharmaceutical composition for the prevention or treatment of cancer is provided, comprising as an active ingredient a compound represented by Formula I or Formula IV, or a pharmaceutically acceptable salt thereof.

According to another aspect of the present invention, a dietary supplement composition for the prevention or amelioration of cancer is provided, comprising as an active ingredient a compound represented by Formula I or Formula IV, or a pharmaceutically acceptable salt thereof.

Furthermore, the present invention provides a method for the prevention or treatment of cancer, comprising the step of administering the pharmaceutical composition in a therapeutically effective amount to a subject in need thereof.

The present invention also provides a method for the prevention or treatment of cancer, comprising the step of administering a compound represented by Formula I or Formula IV, or a pharmaceutically acceptable salt thereof, in a therapeutically effective amount to a subject in need thereof.

Furthermore, the present invention provides a use of the compound represented by Formula I or Formula IV, or a pharmaceutically acceptable salt thereof, for the prevention or treatment of cancer.

### ADVANTAGEOUS EFFECT

The novel quinoxaline compound according to the present invention, or a pharmaceutically acceptable salt thereof, exhibits high inhibitory activity against RET kinase and its mutations, and thus can be usefully used as a pharmaceutical composition for the prevention or treatment of cancer, or as a dietary supplement composition for the prevention or amelioration of cancer.

### BRIEF DESCRIPTION OF THE DRAWINGS

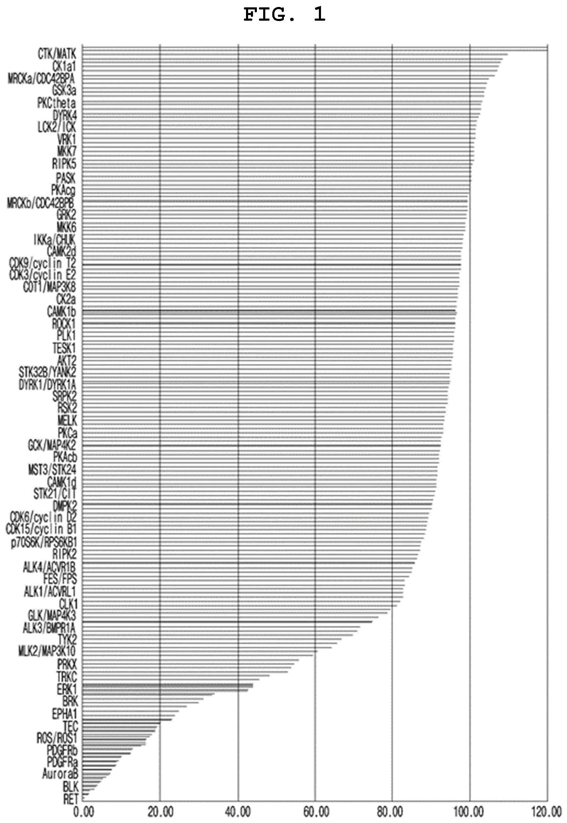

FIG. 1 is a diagram showing the enzymatic inhibitory activity of the example compounds against various kinases.

### DESCRIPTION OF THE PREFERRED EMBODIMENTS

Hereinafter, the present invention is described in detail.

The compounds of the present invention are provided in accordance with Aspects 1 through 4.

### Aspect 1:

In a first embodiment of the present invention,
the compound according to the present invention may be a compound represented by Formula I or Formula IV below.

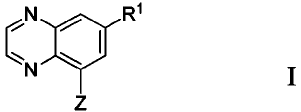

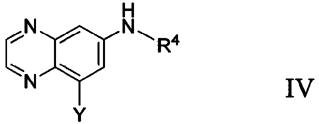

In Formulae I and IV above,
Z is 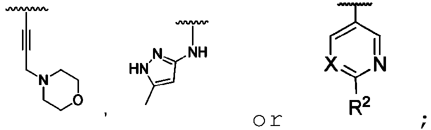
X is CH or N;
R¹ is NO₂ or -NR^{a}R^{b},
wherein each of Ra and Rb is independently H, C₁₋₁₀ alkyl, or 5-6 membered heteroarylcarbonyl;
R² is halogen or 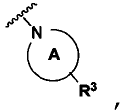
wherein ring A is a 4-6 membered heterocycloalkyl comprising at least one N atom at the point of attachment;
R³ is H, C₁₋₁₀ alkyl, C₁₋₁₀ alkylsulfonyl, C₁₋₃ alkyl substituted with unsubstituted or substituted phenyl, unsubstituted or substituted phenyloxy, unsubstituted or substituted phenyl-CONH-, 5-6 membered heteroaryl, or 6 membered heterocycloalkyl,
wherein the substituted phenyl is each independently substituted with one or more halogens;
Y is halogen; and
R⁴ is C₁₋₁₀ alkyl or 5-6 membered heteroarylcarbonyl.

In a second embodiment according to the first embodiment, the compound may be represented by Formula I.

In a third embodiment according to each of the preceding embodiments, Z may be

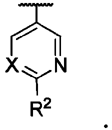

In a fourth embodiment according to the first embodiment, the compound may be represented by Formula IV.

In a fifth embodiment according to each of the preceding embodiments, R^{a} is H, and R^{b} is H, C₁₋₆ alkyl, or 5-6 membered heteroarylcarbonyl; or
each of R^{a} and R^{b} is independently C₁₋₆ alkyl.

In a sixth embodiment according to each of the preceding embodiments, R^{a} is H, and R^{b} is H, C₁₋₆ alkyl, or 5-6 membered heteroarylcarbonyl comprising 1-2 heteroatoms of N or O; or
each of Ra and Rb is independently C1-6 alkyl.

In a seventh embodiment according to each of the preceding embodiments, R^{a} is H, and R^{b} is H, C1-6 alkyl, or 5-6 membered heteroarylcarbonyl comprising 1 heteroatom of N or O; or
each of Ra and Rb is independently C1-6 alkyl.

In an eighth embodiment according to each of the preceding embodiments, R^{a} is H, and R^{b} is H, C₁₋₄ alkyl, furanylcarbonyl, or pyridylcarbonyl; or
each of R^{a} and R^{b} is independently C₁₋₄ alkyl.

In a ninth embodiment according to each of the preceding embodiments, ring A is a 4-6 membered heterocycloalkyl optionally further comprising 1-2 or 1 additional N heteroatoms, or optionally further comprising 1 additional O heteroatom.

In a tenth embodiment according to each of the preceding embodiments, R³ is H, C₁₋₆ alkyl, C₁₋₆ alkylsulfonyl, C₁₋₃ alkyl substituted with substituted phenyl, substituted phenyloxy, substituted phenyl-CONH-, 6 membered heteroaryl comprising 1-2 or 1 N heteroatoms, or morpholinyl,
wherein the substituted phenyl is each independently substituted with 1-2 halogens.

In an eleventh embodiment according to each of the preceding embodiments, R⁴ may be C₁₋₁₀ alkyl or 5-6 membered heteroarylcarbonyl.

In a twelfth embodiment according to each of the preceding embodiments, R⁴ may be C₁₋₅ alkyl, furanylcarbonyl, or pyridylcarbonyl.

### Second Aspect:

In a first embodiment, the compound of Formula I or Formula IV is defined as follows.
X is CH or N;
R¹ is NO₂ or -NR^{a}R^{b},
wherein R^{a} is H or C₁₋₄ alkyl,
and R^{b} is H, C₁₋₄ alkyl, furanylcarbonyl, or pyridylcarbonyl;
ring A is a 4-6 membered heterocycloalkyl optionally further comprising 1 additional heteroatom selected from N and O together with the N atom at the point of attachment; and
R³ is H, C₁₋₃ alkyl, C₁₋₃ alkylsulfonyl, phenylmethyl substituted with 1 halogen, phenyloxy substituted with 1 halogen, phenyl-CONH- substituted with 1 halogen, pyridyl, or morpholinyl.

In a second embodiment according to the first embodiment, the compound may be represented by Formula I.

In a third embodiment according to each of the preceding embodiments, Z may be

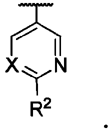

In a fourth embodiment according to the first embodiment, the compound may be represented by Formula IV.

In a fifth embodiment according to each of the preceding embodiments, R^{a} is H, and R^{b} is H, C₁₋₄ alkyl, furanylcarbonyl, or pyridylcarbonyl; or
each of R^{a} and R^{b} is independently C₁₋₄ alkyl.

In a sixth embodiment according to each of the preceding embodiments, R⁴ may be C₁₋₁₀ alkyl or 5-6 membered heteroarylcarbonyl.

In a seventh embodiment according to each of the preceding embodiments, R⁴ may be C₁₋₅ alkyl, furanylcarbonyl, or pyridylcarbonyl.

### Third Aspect:

In a first embodiment, Formula I is defined as follows.
X is CH or N;
R¹ is amino, methylamino, ethylamino, propylamino, isopropylamino, butylamino, isobutylamino, furan-2-yl-CONH-, pyridin-2-yl-CONH-, diethylamino, or nitro;
R² is 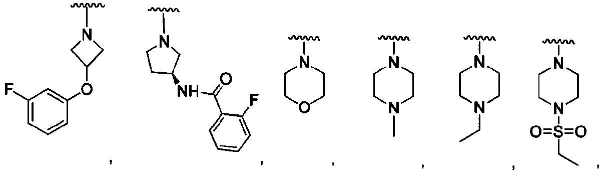 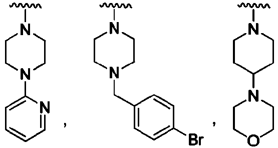 or F.

In a second embodiment according to each of the preceding embodiments, R⁴ may be C₁₋₁₀ alkyl or 5-6 membered heteroarylcarbonyl.

In a third embodiment according to each of the preceding embodiments, R⁴ may be C₁₋₅ alkyl, furanylcarbonyl, or pyridylcarbonyl.

### Fourth Aspect:

The compound represented by Formula I may be any one selected from the group consisting of the following compounds.
<1> 8-(6-(3-(3-fluorophenoxy)azetidin-1-yl)pyridin-3-yl)quinoxalin-6-amine;
<2> 8-(6-(3-(3-fluorophenoxy)azetidin-1-yl)pyridin-3-yl)-N-methylquinoxalin-6-amine;
<3> N-ethyl-8-(6-(3-(3-fluorophenoxy)azetidin-1-yl)pyridin-3-yl)quinoxalin-6-amine;
<4> 8-(6-(3-(3-fluorophenoxy)azetidin-1-yl)pyridin-3-yl)-N-propylquinoxalin-6-amine;
<5> 8-(6-(3-(3-fluorophenoxy)azetidin-1-yl)pyridin-3-yl)-N-isopropylquinoxalin-6-amine;
<6> N-butyl-8-(6-(3-(3-fluorophenoxy)azetidin-1-yl)pyridin-3-yl)quinoxalin-6-amine;
<7> 8-(6-(3-(3-fluorophenoxy)azetidin-1-yl)pyridin-3-yl)-N-isobutylquinoxalin-6-amine;
<8> N-(8-(6-(3-(3-fluorophenoxy)azetidin-1-yl)pyridin-3-yl)quinoxalin-6-yl)furan-2-carboxamide;
<9> N-(8-(6-(3-(3-fluorophenoxy)azetidin-1-yl)pyridin-3-yl)quinoxalin-6-yl)picolinamide;
<10> (S)-N-(1-(5-(7-aminoquinoxalin-5-yl)pyridin-2-yl)pyrrolidin-3-yl)-2-fluorobenzamide;
<11> (S)-2-fluoro-N-(1-(5-(7-(methylamino)quinoxalin-5-yl)pyridin-2-yl)pyrrolidin-3-yl)benzamide;
<12> (S)-N-(1-(5-(7-(ethylamino)quinoxalin-5-yl)pyridin-2-yl)pyrrolidin-3-yl)-2-fluorobenzamide;
<13> (S)-2-fluoro-N-(1-(5-(7-(propylamino)quinoxalin-5-yl)pyridin-2-yl)pyrrolidin-3-yl)benzamide;
<14> (S)-2-fluoro-N-(1-(5-(7-(isopropylamino)quinoxalin-5-yl)pyridin-2-yl)pyrrolidin-3-yl)benzamide;
<15> (S)-N-(1-(5-(7-(butylamino)quinoxalin-5-yl)pyridin-2-yl)pyrrolidin-3-yl)-2-fluorobenzamide;
<16> (S)-2-fluoro-N-(1-(5-(7-(isobutylamino)quinoxalin-5-yl)pyridin-2-yl)pyrrolidin-3-yl)benzamide;
<17> (S)-N-(8-(6-(3-(2-fluorobenzamido)pyrrolidin-1-yl)pyridin-3-yl)quinoxalin-6-yl)furan-2-carboxamide;
<18> (S)-N-(8-(6-(3-(2-fluorobenzamido)pyrrolidin-1-yl)pyridin-3-yl)quinoxalin-6-yl)picolinamide;
<19> 8-(6-morpholinopyridin-3-yl)quinoxalin-6-amine;
<20> 8-(6-(4-methylpiperazin-1-yl)pyridin-3-yl)quinoxalin-6-amine;
<21> 8-(6-(4-ethylpiperazin-1-yl)pyridin-3-yl)quinoxalin-6-amine;
<22> 8-(6-(4-(ethylsulfonyl)piperazin-1-yl)pyridin-3-yl)quinoxalin-6-amine;
<23> 8-(6-(4-(pyridin-2-yl)piperazin-1-yl)pyridin-3-yl)quinoxalin-6-amine;
<24> 8-(6-(4-(4-bromobenzyl)piperazin-1-yl)pyridin-3-yl)quinoxalin-6-amine;
<25> 8-(6-(4-morpholinopiperidin-1-yl)pyridin-3-yl)quinoxalin-6-amine;
<26> 8-(6-(4-(4-bromobenzyl)piperazin-1-yl)pyridin-3-yl)-N-ethylquinoxalin-6-amine;
<27> 5-(6-(3-(3-fluorophenoxy)azetidin-1-yl)pyridin-3-yl)-7-nitroquinoxaline;
<28> N,N-diethyl-8-(6-(3-(3-fluorophenoxy)azetidin-1-yl)pyridin-3-yl)quinoxalin-6-amine;
<29> 8-(2-(3-(3-fluorophenoxy)azetidin-1-yl)pyrimidin-5-yl)quinoxalin-6-amine;
<30> (S)-N-(1-(5-(7-aminoquinoxalin-5-yl)pyrimidin-2-yl)pyrrolidin-3-yl)-2-fluorobenzamide;
<31> N-ethyl-8-(3-morpholinoprop-1-yn-1-yl)quinoxalin-6-amine; and
<32> N7-ethyl-N5-(5-methyl-1H-pyrazol-3-yl)quinoxaline-5,7-diamine.

The compound represented by Formula IV may be any one selected from the group consisting of the following compounds.
<1> 8-bromo-N-butylquinoxalin-6-amine;
<2> 8-bromo-N-isobutylquinoxalin-6-amine;
<3> 8-(6-fluoropyridin-3-yl)quinoxalin-6-amine;
<4> 8-(6-fluoropyridin-3-yl)-N-methylquinoxalin-6-amine;
<5> N-ethyl-8-(6-fluoropyridin-3-yl)quinoxalin-6-amine;
<6> 8-(6-fluoropyridin-3-yl)-N-propylquinoxalin-6-amine;
<7> 8-(6-fluoropyridin-3-yl)-N-isopropylquinoxalin-6-amine;
<8> N-butyl-8-(6-fluoropyridin-3-yl)quinoxalin-6-amine;
<9> 8-(6-fluoropyridin-3-yl)-N-isobutylquinoxalin-6-amine;
<10> N-(8-bromoquinoxalin-6-yl)furan-2-carboxamide;
<11> N-(8-bromoquinoxalin-6-yl)picolinamide;
<12> N-(8-(6-fluoropyridin-3-yl)quinoxalin-6-yl)furan-2-carboxamide;
<13> N-(8-(6-fluoropyridin-3-yl)quinoxalin-6-yl)picolinamide;
<14> 5-(6-fluoropyridin-3-yl)-7-nitroquinoxaline;
<15> 8-(2-chloropyrimidin-5-yl)quinoxalin-6-amine;
<16> 8-bromo-N-ethylquinoxalin-6-amine;
<17> 8-bromo-N-propylquinoxalin-6-amine; and
<18> 8-bromo-N-isopropylquinoxalin-6-amine.

As used herein, the term "alkyl," unless otherwise specified, includes straight-chain or branched saturated hydrocarbon residues. For example, "C1-6 alkyl" refers to an alkyl having a backbone of 1 to 6 carbon atoms. Specifically, C1-6 alkyl includes methyl, ethyl, n-propyl, i-propyl, n-butyl, i-butyl, t-butyl, n-pentyl, i-pentyl, t-pentyl, sec-pentyl, neopentyl, hexyl, and the like.

The term "cycloalkyl," unless otherwise specified, refers to a cyclic hydrocarbon residue forming a ring as a saturated hydrocarbon residue, and "3-6 membered cycloalkyl" refers to a cyclic hydrocarbon residue comprising 3 to 6 carbon atoms as ring atoms.

The term "heterocycloalkyl," unless otherwise specified, refers to a monovalent saturated residue consisting of 1 to 3 rings comprising one or more (e.g., 1-5, 1-4, 1-3, 1-2, or 1) heteroatoms selected from N, O, or S. It may be bicyclic or tricyclic comprising 2 or 3 rings, wherein the 2 or 3 rings may be bridged, fused, or spiro heterocycloalkyl. When consisting of a plurality of rings, the heteroatoms may be present in all rings or only in some of the rings.

The term "aryl" refers to an aromatic radical in which a single ring or 2 or 3 hydrocarbon aromatic rings are fused, and "C₆₋₁₀ aryl" refers to an aromatic ring compound comprising 6 to 10 carbon atoms, and includes phenyl or naphthyl.

The term "heteroaryl," unless otherwise specified, refers to a single ring or an aromatic radical in which 2 or 3 rings are fused, having one or more aromatic rings (the remaining ring atoms being C) containing one or more (e.g., 1-5, 1-4, 1-3, 1-2, or 1) heteroatoms selected from N, O, or S as ring atoms. When consisting of a plurality of rings, the heteroatoms may be present in all rings or only in some of the rings.

The term "halogen" refers to a halogen atom such as F, Cl, Br, or I, and when substituted with "halogen," it means substituted with one or more halogen atoms. In this case, the number of substituted halogen atoms may be selected within the range of 1 to 5.

The term "pharmaceutically acceptable salt" refers to a pharmaceutically acceptable salt formed by ionic bonding with a compound when the compound contains a carboxylic acid or amine functional group.

The compound represented by Formula I or Formula IV of the present invention may be used in the form of a pharmaceutically acceptable salt, and acid addition salts formed by pharmaceutically acceptable free acids are useful as such salts. Acid addition salts are obtained from inorganic acids such as hydrochloric acid, nitric acid, phosphoric acid, sulfuric acid, hydrobromic acid, hydroiodic acid, nitrous acid, and phosphorous acid; non-toxic organic acids such as aliphatic mono- and dicarboxylates, phenyl-substituted alkanoates, hydroxy alkanoates and alkanedioates, aromatic acids, and aliphatic and aromatic sulfonic acids; and organic acids such as trifluoroacetic acid, acetate, benzoic acid, citric acid, lactic acid, maleic acid, gluconic acid, methanesulfonic acid, 4-toluenesulfonic acid, tartaric acid, and fumaric acid. Such pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate, mandelate, and the like.

The acid addition salt according to the present invention may be prepared by a conventional method, for example, by dissolving a derivative of Formula I or Formula IV in an organic solvent such as methanol, ethanol, acetone, methylene chloride, acetonitrile, or the like, adding an organic or inorganic acid, and filtering and drying the resulting precipitate; or by distilling the solvent and excess acid under reduced pressure, drying, and crystallizing in an organic solvent.

In addition, pharmaceutically acceptable metal salts may be prepared using a base. Alkali metal or alkaline earth metal salts are obtained, for example, by dissolving the compound in an excess of an alkali metal hydroxide or alkaline earth metal hydroxide solution, filtering off the insoluble compound salt, and evaporating and drying the filtrate. In this case, it is pharmaceutically suitable to prepare sodium, potassium, or calcium salts as the metal salts. In addition, the corresponding salts are obtained by reacting the alkali metal or alkaline earth metal salt with a suitable silver salt (e.g., silver nitrate).

Another aspect of the present invention provides a method of preparing a compound of Formula IA, comprising reacting a compound of Formula II with a compound of Formula III as shown in Reaction Scheme I below. 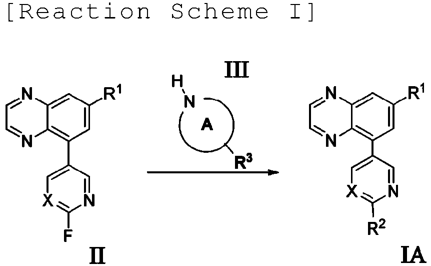 wherein X, R¹, R², ring A, and R³ are as defined in Formula I.

In the above reaction scheme, the reaction of Formula II with Formula III is a nucleophilic substitution reaction, and the reaction conditions are carried out under conventional organic solvent conditions.

The reaction temperature is not particularly limited, but may be carried out in the range of 0 to 100°C or 10 to 50°C, and may be carried out at room temperature.

Another aspect of the present invention provides a pharmaceutical composition for the prevention or treatment of cancer, comprising as an active ingredient a compound represented by Formula I or Formula IV, or a pharmaceutically acceptable salt thereof.

In this case, the cancer is one or more selected from the group consisting of pseudomyxoma, intrahepatic cholangiocarcinoma, hepatoblastoma, hepatocellular carcinoma, thyroid cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell tumor, male breast cancer, brain cancer, pituitary adenoma, multiple myeloma, gallbladder cancer, cholangiocarcinoma, colorectal cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, tongue cancer, astrocytoma, small intestine cancer, meningioma, esophageal cancer, glioma, renal pelvis cancer, renal cancer, cardiac cancer, duodenal cancer, malignant soft tissue cancer, malignant bone cancer, malignant lymphoma, malignant mesothelioma, malignant melanoma, ocular cancer, vulvar cancer, ureteral cancer, urethral cancer, cancer of unknown primary, gastric lymphoma, gastric cancer, gastric carcinoid tumor, gastrointestinal stromal tumor, Wilms tumor, breast cancer, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, vaginal cancer, spinal cord cancer, acoustic neuroma, pancreatic cancer, salivary gland cancer, Kaposi's sarcoma, Paget's disease, tonsillar cancer, squamous cell carcinoma, lung cancer, skin cancer, anal cancer, rhabdomyosarcoma, laryngeal cancer, pleural cancer, and thymic cancer.

In addition, the compound or a pharmaceutically acceptable salt thereof can inhibit RET and RET mutations, and through such inhibition, has activity for the prevention, amelioration, or treatment of cancer, wherein the RET mutations may include point mutations, fusion mutations, and multiple mutations. The point mutations may include RET(G810R), RET(V804M), and RET(G810S/M918T), the fusion mutations may include KIF5B-RET, RET-CCDC6, and RET-NCOA4, and the multiple mutations may include KIF5B-RET, KIF5B-RET(V804M), KIF5B-RET(G810R), RET(V804M)-KIF5B, and RET(G810S/M918T). In this case, the cancer is the same as described above.

In addition, the compound or a pharmaceutically acceptable salt thereof may selectively inhibit tyrosine kinase, wherein the kinase is a receptor tyrosine kinase, and in one embodiment, the kinase may include RET, c-Kit (Tyrosine-protein kinase KIT, CD117), DDR1 (Discoidin domain receptor family, member 1), FLT3 (fms related receptor tyrosine kinase 3), VEGFR3 (Vascular endothelial growth factor receptor 3), EPHB1 (Ephrin type-B receptor 1), and the like.

The compound or a pharmaceutically acceptable salt thereof has growth inhibitory activity against mutant cancer cells expressing RET and RET mutations as described above, and in one embodiment, may be cancer cells expressing one of G810R, V804M, V804R, or the like.

In addition, the pharmaceutical composition for the prevention or treatment of cancer, comprising as an active ingredient a compound represented by Formula I or Formula IV, an optical isomer thereof, or a pharmaceutically acceptable salt thereof, may be administered as an individual therapeutic agent or in combination with other anticancer agents in use.

In addition, the pharmaceutical composition for the prevention or treatment of cancer, comprising as an active ingredient a compound represented by Formula I or Formula IV, an optical isomer thereof, or a pharmaceutically acceptable salt thereof, may enhance anticancer effects when administered in combination with an anticancer agent.

The compound represented by Formula I or Formula IV, or a pharmaceutically acceptable salt thereof, may be administered in various oral and parenteral dosage forms for clinical administration. When formulated, it is prepared using diluents or excipients such as fillers, extenders, binders, wetting agents, disintegrants, and surfactants that are commonly used. Solid preparations for oral administration include tablets, pills, powders, granules, and capsules, and such solid preparations are prepared by mixing one or more compounds with at least one excipient, such as starch, calcium carbonate, sucrose, lactose, gelatin, or the like. In addition to simple excipients, lubricants such as magnesium stearate and talc are also used. Liquid preparations for oral administration include suspensions, oral solutions, emulsions, and syrups, and in addition to water and liquid paraffin, which are commonly used simple diluents, various excipients such as wetting agents, sweeteners, flavoring agents, and preservatives may be included. Preparations for parenteral administration include sterile aqueous solutions, non-aqueous solutions, suspensions, and emulsions. Non-aqueous solvents and suspending agents that may be used include propylene glycol, polyethylene glycol, vegetable oils such as olive oil, and injectable esters such as ethyl oleate.

The pharmaceutical composition comprising as an active ingredient a compound represented by Formula I or Formula IV, or a pharmaceutically acceptable salt thereof, may be administered parenterally, and parenteral administration is by means of subcutaneous injection, intravenous injection, intramuscular injection, or intrathoracic injection.

In this case, to formulate into a dosage form for parenteral administration, the compound represented by Formula I or Formula IV, or a pharmaceutically acceptable salt thereof, may be mixed with a stabilizer or buffer in water to prepare a solution or suspension, which may then be prepared in ampoule or vial unit dosage forms. The composition may be sterilized and/or may contain adjuvants such as preservatives, stabilizers, wetting agents or emulsification promoters, salts and/or buffers for osmotic pressure regulation, and other therapeutically useful substances, and may be formulated according to conventional methods of mixing, granulation, or coating.

Dosage forms for oral administration include, for example, tablets, pills, hard/soft capsules, solutions, suspensions, emulsions, syrups, granules, elixirs, and troches, and these dosage forms contain, in addition to the active ingredient, diluents (e.g., lactose, dextrose, sucrose, mannitol, sorbitol, cellulose, and/or glycine) and lubricants (e.g., silica, talc, stearic acid and its magnesium or calcium salts, and/or polyethylene glycol). Tablets may contain binders such as magnesium aluminum silicate, starch paste, gelatin, methylcellulose, sodium carboxymethylcellulose, and/or polyvinylpyrrolidine, and may optionally contain disintegrants or effervescent mixtures such as starch, agar, alginic acid or its sodium salt, and/or absorbents, colorants, flavoring agents, and sweeteners.

Another aspect of the present invention provides a dietary supplement for the prevention or amelioration of cancer, comprising as an active ingredient a compound represented by Formula I or Formula IV, or a pharmaceutically acceptable salt thereof. In this case, the cancer is the same as described above.

The compound represented by Formula I or Formula IV according to the present invention may be added directly to food or used together with other food or food ingredients, and may be appropriately used according to conventional methods. The amount of the active ingredient to be mixed may be suitably determined according to the purpose of use (prevention or amelioration). In general, the amount of the compound in health food may be added in an amount of 0.1 to 90 parts by weight based on the total food weight. However, in the case of long-term intake for the purpose of health and hygiene or health management, the amount may be below the above range, and since there is no problem in terms of safety, the active ingredient may also be used in an amount exceeding the above range.

Another aspect of the present invention provides a method for the prevention, amelioration, or treatment of cancer, comprising the step of administering to a subject in need thereof a pharmaceutical composition or dietary supplement comprising as an active ingredient a compound represented by Formula I or Formula IV, or a pharmaceutically acceptable salt thereof.

Another aspect of the present invention provides a use of the compound represented by Formula I, or a pharmaceutically acceptable salt thereof, for the prevention, amelioration, or treatment of cancer.

The compound represented by Formula I or Formula IV of the present invention, or a pharmaceutically acceptable salt or pharmaceutical composition thereof, is administered in a "pharmaceutically effective amount." As used herein, the term "pharmaceutically effective amount" refers to an amount sufficient to treat a disease at a reasonable benefit/risk ratio applicable to medical treatment or amelioration, and the effective dose level may be determined according to factors including the type and severity of the subject, age, sex, drug activity, sensitivity to the drug, time of administration, route of administration and excretion rate, duration of treatment, drugs used concurrently, and other factors well known in the medical field. For example, effective amounts of 0.001 mg/kg to 1000 mg/kg, 0.01 mg/kg to 100 mg/kg, 0.1 to 20 mg/kg, or 0.1 to 500 mg/kg are included. The amount of the pharmaceutical composition of the present invention may be selected and practiced by those skilled in the art within an appropriate range.

Hereinafter, specific examples for the compound according to the present invention and the preparation method therefor are presented. However, the following examples are provided only for understanding the invention and should not be construed as limiting the present invention.

### Preparation Example: Preparation of Intermediate Compounds

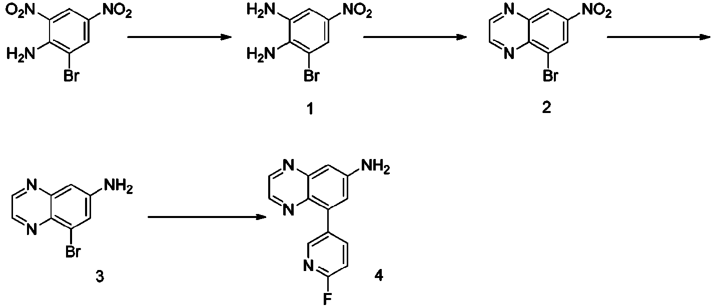

### Intermediate Compound 1: Preparation of 3-bromo-5-nitrobenzene-1,2-diamine

To a solution of 2-bromo-4,6-dinitroanilne (1 eq., 10 g) dissolved in ethanol was added 5% Pd/C (500 mg), followed by the addition of hydrazine monohydrate (5 eq.) at room temperature. The reaction mixture was then stirred at room temperature for 1 hour and monitored by TLC. After completion of the reaction, the reaction mixture was filtered through Celite. The filtrate was cooled with water and extracted with ethyl acetate. The organic layer was washed with brine, dried over sodium sulfate, and evaporated under reduced pressure to give a brown crude material. The crude material was used in the next step without purification. Brown solid (6.5 g, crude).

### Intermediate Compound 2: Preparation of 5-bromo-7-nitroquinoxaline

To a solution of Intermediate Compound 1 (1 eq.) dissolved in water (20 vol) was added glyoxal (7.8 mL), and the resulting reaction mixture was stirred at 100°C for 16 hours. The progress of the reaction was monitored by TLC (eluent = n-hexane/ethyl acetate: 8/2). After completion of the reaction, the mixture was cooled to room temperature and extracted with ethyl acetate. The organic layer was washed with brine, dried over sodium sulfate, and evaporated under reduced pressure to give a crude material. The crude material was purified by silica gel column chromatography eluting with ethyl acetate/hexane to give 5-bromo-7-nitroquinoxaline (yellow solid; 46% yield).

### Intermediate Compound 3: Preparation of 8-bromoquinoxalin-6-amine

To a solution of Intermediate Compound 2 (1 eq.) dissolved in acetonitrile:water (1:0.3) was added NiCl2·6H2O (0.2 eq.), followed by the portionwise addition of NaBH4 (4 eq.) at room temperature. The reaction mixture was then stirred at room temperature for 1 hour and monitored by TLC (eluent = n-hexane/ethyl acetate: 6/4). After completion of the reaction, water was added to the reaction mixture and extracted with ethyl acetate. The organic layer was washed with brine, dried over sodium sulfate, and evaporated under reduced pressure to give a crude material. The crude material was purified by silica gel column chromatography eluting with ethyl acetate/hexane to give 8-bromoquinoxalin-6-amine (yellow solid; 58% yield).

### Intermediate Compound 4: Preparation of 8-(6-fluoropyridin-3-yl)quinoxalin-6-amine

To a degassed solution of Intermediate Compound 3 (1 eq.), 2-fluoro-5-(4,4,5,5-tetramethyl-1,3,2-dioxaborolan-2-yl)pyridine (1.3 eq.), and K2CO3 (2 eq.) in 1,4-dioxane:water (1:0.3) was added Pd(dppf)Cl2·DCM complex (0.04 eq.), and the mixture was stirred at 100°C for 16 hours. The reaction was monitored by TLC (eluent = n-hexane/ethyl acetate: 6/4). After completion of the reaction, the reaction mixture was filtered through Celite. The filtrate was treated with water and extracted with ethyl acetate. The organic layer was dried over sodium sulfate and evaporated under reduced pressure to give a crude material. The crude material was purified by silica gel column chromatography eluting with ethyl acetate/hexane to give 8-(6-fluoropyridin-3-yl)quinoxalin-6-amine (yellow solid; 76% yield).

### Intermediate Compound 5: Preparation of 8-(6-fluoropyridin-3-yl)-N-methylquinoxalin-6-amine

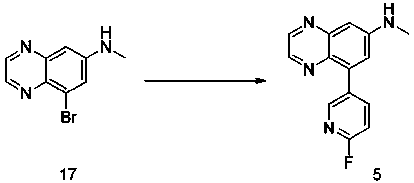

Intermediate Compound 5 was prepared in a similar manner to the preparation of Intermediate Compound 4 (yellow solid; 70% yield).

Intermediate Compounds 6 to 10 were additionally prepared in a similar manner.

### Intermediate Compound 11: Preparation of N-(8-bromoquinoxalin-6-yl)furan-2-carboxamide

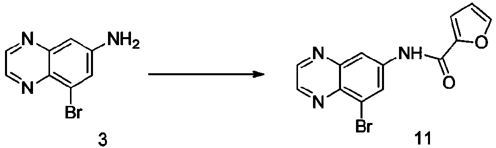

To a mixture of Intermediate Compound 3 (1 eq.), 2-furoic acid (2 eq.) or picolinic acid, and TEA (3 eq.) was added DCM, followed by the addition of T3P (2 eq.), and the reaction mixture was stirred at room temperature for 32 hours. The reaction was monitored by TLC (eluent = n-hexane/ethyl acetate: 6/4). After completion of the reaction, water was added to the reaction mixture and extracted with DCM. The organic layer was dried over sodium sulfate and evaporated under reduced pressure to give a crude material. The crude material was purified by silica gel column chromatography eluting with ethyl acetate/hexane to give a solid product (yellow solid; 59% yield).

Intermediate Compound 12 was additionally prepared in a similar manner.

### Intermediate Compound 13: Preparation of N-(8-(6-fluoropyridin-3-yl)quinoxalin-6-yl)furan-2-carboxamide

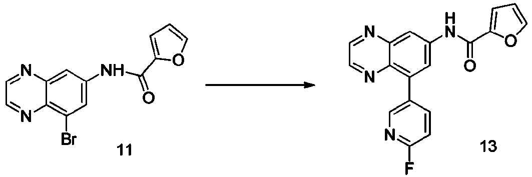

**Intermediate Compound 13 was prepared in a similar manner to the preparation of Intermediate Compound 5 (yellow solid; 58% yield).**

Intermediate Compound 14 was additionally prepared in a similar manner.

### Intermediate Compound 15: Preparation of 5-(6-fluoropyridin-3-yl)-7-nitroquinoxaline

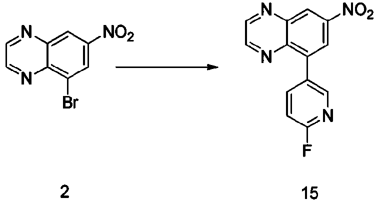

Intermediate Compound 15 was prepared in a similar manner to the preparation of Intermediate Compound 4 (yellow solid; 64% yield).

### Intermediate Compound 16: Preparation of 8-(2-chloropyrimidin-5-yl)quinoxalin-6-amine

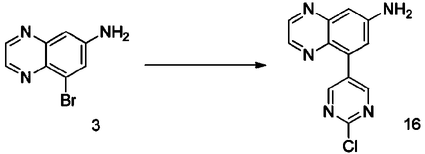

Intermediate Compound 16 was prepared in a similar manner to the preparation of Intermediate Compound 4 (yellow solid; 35% yield).

### Intermediate Compound 17: Preparation of 8-bromo-N-methylquinoxalin-6-amine

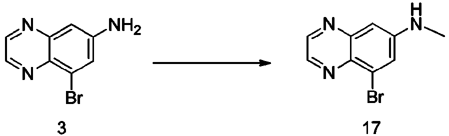

To a solution of Intermediate Compound 3 (1 eq.) dissolved in DMF was added K2CO3 (2 eq.), followed by the addition of alkyl iodide (1.5 eq.) at room temperature. The reaction mixture was then stirred at 80°C for 16 hours and monitored by TLC (eluent = n-hexane/ethyl acetate: 8/2). A new spot appeared along with the starting material on TLC, and additional alkyl iodide (1.5 eq.) was added and stirred for 16 hours. After completion of the reaction, water was added to the reaction mixture and extracted with ethyl acetate. The organic layer was washed with brine, dried over sodium sulfate, and evaporated under reduced pressure to give a crude material. The crude material was purified by silica gel column chromatography eluting with ethyl acetate/hexane to give 8-bromo-N-methylquinoxalin-6-amine (yellow solid; 43% yield).

Intermediate Compounds 18 to 22 were additionally prepared in a similar manner.

The structures of the intermediate compounds prepared in the Preparation Example are summarized in Table 1 below.

**[Table 1]**

| Number | Name | Structure | NMR |
|---|---|---|---|
| 1 | 3-bromo-5-nitrobenzene-1,2-diamine | 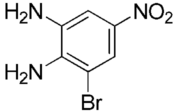 | - |
| 2 | 5-bromo-7-nitroquinoxaline | 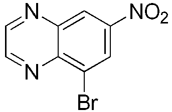 | 1H NMR (600 MHz, CDCl3) δ 9.25 (d, J = 1.7 Hz, 1H), 9.22 (d, J = 1.7 Hz, 1H), 8.93 (d, J = 2.4 Hz, 1H), 8.88 (d, J = 2.4Hz, 1H) |
| 3 | 8-bromoquinoxal in-6-amine | 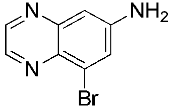 | 1H NMR (600 MHz, CDCl3) δ 8.69 (d, J = 1.8 Hz, 1H), 8.66 (d, J = 1.8 Hz, 1H), 7.56 (d, J = 2.4 Hz, 1H), 7.13 (d, J = 2.4 Hz, 1H), 4.25 (s, 2H) |
| 4 | 8-(6-fluoropyridin -3-yl)quinoxalin -6-amine | 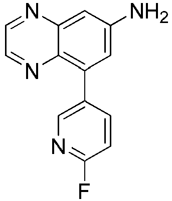 | 1H NMR (600 MHz, CDCl3) δ 8.66 (d, J = 1.8 Hz, 1H), 8.46 (d, J = 1.8 Hz, 1H), 8.43 (d, J = 2.4 Hz, 1H), 8.24 - 8.20 (m, 1H), 7.33 (d, J = 2.4 Hz, 1H), 7.15 (dd, J = 8.4, 2.4 Hz, 1H), 7.00 (d, J = 2.4 Hz, 1H), 6.18 (s, 2H). |
| 5 | 8-(6-fluoropyridin -3-yl)-N-methylquinoxa lin-6-amine | 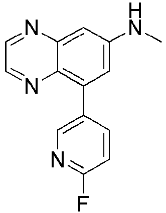 | 1H NMR (600 MHz, CDCl3) δ 8.68 (d, J = 1.8 Hz, 1H), 8.52 (d, J = 1.8 Hz, 1H), 8.45 - 8.43 (m, 1H), 8.14 (td, J = 8.1 Hz, 1H), 7.15 (d, J = 2.6 Hz, 1H), 7.08 - 7.05 (m, 1H), 7.03 (d, J = 2.6 Hz, 1H), 4.42 (bs, 1H), 3.04 (d, J = 5.1 Hz, 3H). 13C NMR (150 MHz, CDCl3) δ 149.58, 148.23, 145.98, 145.18, 143.43, 140.14, 136.72, 135.80, 131.84, 122.06, 108.84, 108.59, 103.81, 30.50 |
| 6 | N-ethyl-8-(6-fluoropyridin -3-yl)quinoxalin -6-amine | 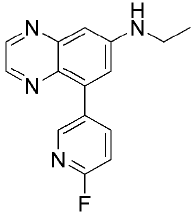 | 1H NMR (600 MHz, CDCl3) δ 1H NMR (600 MHz, CDCl3) δ 8.67 (d, J = 1.8 Hz, 1H), 8.50 (d, J = 1.8 Hz, 1H), 8.44 (d, J = 2.4 Hz, 1H), 8.14 (td, J = 8.0 Hz, 1H), 7.14 (d, J = 2.6 Hz, 1H), 7.06 (dd, J = 8.4 Hz, 1H), 7.03 (d, J = 2.6 Hz, 1H), 4.29 (s, 1H), 3.38 - 3.33 (m, 2H), 1.38 (t, J = 7.2 Hz, 3H). 13C NMR (150 MHz, CDCl3) δ 148.67, 148.20, 145.94, 145.13, 143.43, 140.07, 136.75, 135.75, 131.85, 131.82, 122.23, 108.84, 104.03, 38.30, 14.41 |
| 7 | 8- (6-fluoropyridin -3-yl)-N-isopropylquin oxalin-6-amine | 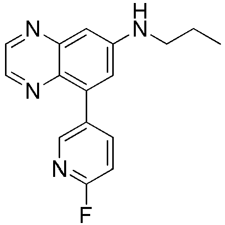 | 1H NMR (600 MHz, CDCl3) δ 8.66 (d, J = 1.9 Hz, 1H), 8.49 (d, J = 1.9 Hz, 1H), 8.44 (d, J = 2.4 Hz, 1H), 8.14 (td, J = 8.1, 2.5 Hz, 1H), 7.14 (d, J = 2.6 Hz, 1H), 7.05 (dd, J = 8.4, 2.8 Hz, 1H), 7.02 (d, J = 2.6 Hz, 1H), 4.40 (s, 1H), 3.28 (q, J = 7.4 Hz, 2H), 1.80 - 1.73 (m, 2H), 1.07 (t, J = 7.4 Hz, 3H) |
| 8 | 8-(6-fluoropyridin -3-yl)-N-isopropylquin oxalin-6-amine | 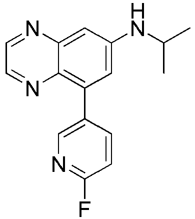 | 1H NMR (600 MHz, CDCl3) δ 8.66 (d, J = 1.7 Hz, 1H), 8.49 (d, J = 1.7 Hz, 1H), 8.44 (d, J = 1.7 Hz, 1H), 8.15 - 8.12 (m, 1H), 7.10 (d, J = 2.5 Hz, 1H), 7.06 (dd, J = 8.4 Hz, 1H), 7.02 (d, J = 2.4 Hz, 1H), 4.20 (d, J = 6.9 Hz, 1H), 3.86 - 3.81 (m, 1H), 1.34 (d, J = 6.3 Hz, 6H) |
| 9 | N-butyl-8-(6-fluoropyridin -3-yl)quinoxalin -6-amine | 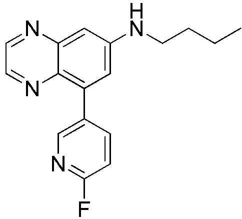 | 1H NMR (600 MHz, CDCl3) δ 8.66 (d, J = 1.8 Hz, 1H), 8.50 (d, J = 1.8 Hz, 1H), 8.44 (d, J = 2.4 Hz, 1H), 8.14 (td, J = 8.0 Hz, 1H), 7.14 (d, J = 3.0 Hz, 1H), 7.06 (dd, J = 8.4, 2.4 Hz, 1H), 7.02 (d, J = 2.4 Hz, 1H), 4.33 (s, 1H), 3.33 - 3.31 (m, 2H), 1.75 - 1.70 (m, 2H), 1.52 - 1.48 (m, 2H), 1.00 (t, J = 7.2 Hz, 3H) |
| 10 | 8-(6-Fluoropyridin -3-yl)-N-isobutylquinoxalin-6-amine | 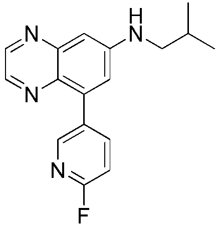 | 1H NMR (600 MHz, CDCl3) δ 8.66 (d, J = 1.8 Hz, 1H), 8.50 (d, J = 1.8 Hz, 1H), 8.45 (d, J = 1.7 Hz, 1H), 8.16 - 8.13 (m, 1H), 7.15 (d, J = 2.4 Hz, 1H), 7.06 (dd, J = 8.4, 2.4 Hz, 1H), 7.02 (d, J = 2.4 Hz, 1H), 4.42 (s, 1H), 3.14 (d, J = 6.6 Hz, 6H), 2.08 - 2.01 (m, 1H), 1.06 (d, J = 6.6 Hz, 6H) |
| 11 | N-(8-Bromoquinoxalin-6-yl)furan-2-carboxamide | 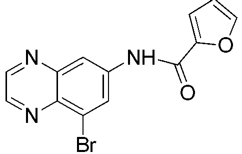 | 1H NMR (600 MHz, CDCl3) δ 9.68 (s, 1H), 8.85 (d, J = 2.8 Hz, 2H), 8.64 (s, 1H), 8.61 (s, 1H), 7.62 (s, 1H), 7.38 (d, J = 3.2 Hz, 1H), 6.60 (s, 1H). 13C NMR (150 MHz, CDCl3 + DMSO-d6) δ = 156.73, 147.35, 145.92, 144.93, 144.27, 143.97, 139.88, 137.71, 127.79, 123.92, 117.36, 115.83, 112.44 |
| 12 | N-(8-Bromoquinoxal in-6-yl)picolinamide | 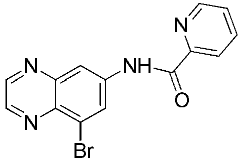 | 1H NMR (600 MHz, CDCl3) δ 10.41 (s, 1H), 8.89 - 8.86 (m, 2H), 8.69 - 8.66 (m, 1H), 8.64 (t, J = 1.8 Hz, 1H), 8.58 (t, J = 1.8 Hz, 1H), 8.36 (dd, J = 7.8, 0.8 Hz, 1H), 7.99 - 7.96 (m, 1H), 7.58 - 7.55 (m, 1H) |
| 13 | N-(8-(6-Fluoropyridin -3-yl)quinoxalin -6-yl)furan-2-carboxamide | 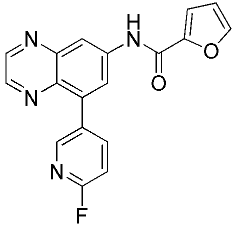 | 1H NMR (600 MHz, DMSO-d6) δ 10.72 (s, 1H), 8.94 (s, 1H), 8.84 (s, 1H), 8.71 (s, 1H), 8.52 (s, 1H), 8.30 (s, 2H), 8.01 (s, 1H), 7.45 (s, 1H), 7.36 (d, J = 7.2 Hz, 1H), 6.76 (s, 1H). 13C NMR (150 MHz, DMSO-d6) δ 157.15, 148.75, 148.65, 147.49, 146.84, 146.65, 144.50, 143.80, 140.04, 137.45, 136.22, 132.12, 125.24, 117.49, 116.22, 112.93, 109.49, 109.24 |
| 14 | N-(8-(6-Fluoropyridin -3-yl)quinoxalin -6-yl)picolinamide | 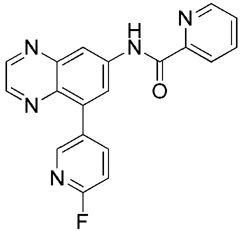 | 1H NMR (600 MHz, CDCl3) δ 10.47 (d, J = 12.8 Hz, 1H), 8.88 (dd, J = 7.2, 1.6 Hz, 1H), 8.78 (s, 1H), 8.70 - 8.67 (m, 1H), 8.62 - 8.61 (m, 1H), 8.57 (dd, J = 5.1, 2.3 Hz, 1H), 8.37 (d, J = 7.8 Hz, 1H), 8.33 (d, J = 2.3 Hz, 1H), 8.23 - 8.19 (m, 1H), 7.99 - 7.96 (m, 1H), 7.57 - 7.55 (m, 1H), 7.10 (dd, J = 8.4, 2.3 Hz, 1H) |
| 15 | 5-(6-Fluoropyridin -3-yl)-7-nitroquinoxaline | 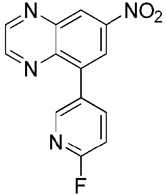 | 1H NMR (600 MHz, CDCl3) δ 9.08 (d, J = 1.8 Hz, 2H), 9.03 (d, J = 1.6 Hz, 1H), 8.62 (d, J = 2.6 Hz, 1H), 8.58 (d, J = 2.4 Hz, 1H), 8.21 - 8.17 (m, 1H), 7.14 (dd, J = 8.4 Hz, 2.8, 1H) |
| 16 | 8-(2-Chloropyrimid in-5-yl)quinoxalin -6-amine | 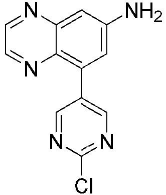 | 1H NMR (600 MHz, CDCl3) δ 9.03 (s, 1H), 8.69 (d, J = 1.2 Hz, 1H), 8.49 (d, J = 1.2 Hz, 1H), 7.42 (d, J = 2.4 Hz, 1H), 7.05 (td, J = 1.8 Hz, 1H), 6.25 (s, 1H) |
| 17 | 8-Bromo-N-methylquinoxalin-6-amine | 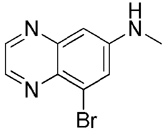 | 1H NMR (600 MHz, CDCl3) δ 8.66 (d, J = 1.8 Hz, 1H), 8.61 (d, J = 1.8 Hz, 1H), 7.49 (d, J = 2.4 Hz, 1H), 6.94 (d, J = 2.4 Hz, 1H), 4.34 (s, 1H), 2.98 (d, J = 5.2 Hz, 3H). 13C NMR (150 MHz, CDCl3) δ 150.14, 146.02, 145.63, 140.73, 135.29, 124.93, 124.51, 103.44, 30.45 |
| 18 | 8-Bromo-N-ethylquinoxalin-6-amine | 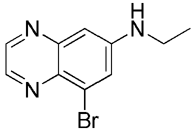 | 1H NMR (600 MHz, CDCl3) δ 8.65 (d, J = 1.9 Hz, 1H), 8.59 (d, J = 1.9 Hz, 1H), 7.47 (d, J = 2.5 Hz, 1H), 6.93 (d, J = 2.5 Hz, 1H), 4.20 (s, 1H), 3.29 (m, 2H), 1.34 (t, J = 7.2 Hz, 3H). 13C NMR (150 MHz, CDCl3) δ 149.23, 146.01, 145.59, 140.66, 135.24, 125.08, 124.52, 103.67, 38.28, 14.33 |
| 19 | 8-Bromo-N-propylquinoxalin-6-amine | 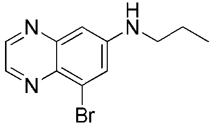 | 1H NMR (600 MHz, CDCl3) δ 8.65 (d, J = 1.2 Hz, 1H), 8.59 (d, J = 1.8 Hz, 1H), 7.49 (d, J = 2.4 Hz, 1H), 6.93 (d, J = 2.4 Hz, 1H), 4.27 (s, 1H), 3.24 - 3.21 (m, 2H), 1.76 - 1.70 (m, 2H), 1.05 (t, J = 7.2 Hz, 3H) |
| 20 | 8-Bromo-N-isopropylquin oxalin-6-amine | 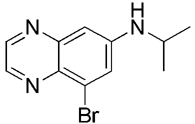 | 1H NMR (600 MHz, CDCl3) δ 8.64 (s, 1H), 8.59 (d, J = 1.8 Hz, 1H), 7.45 (d, J = 2.5 Hz, 1H), 6.93 (d, J =2.5 Hz, 1H), 4.12 (s, 1H), 3.80 - 3.73 (m, 1H), 1.31 (d, J = 6.3 Hz, 6H) |
| 21 | 8-Bromo-N-butylquinoxalin-6-amine | 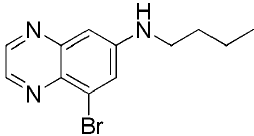 | 1H NMR (600 MHz, CDCl3) δ 8.64 (d, J = 1.8 Hz, 1H), 8.59 (d, J = 1.8 Hz, 1H), 7.48 (d, J = 2.4 Hz, 1H), 6.93 (d, J = 3.0 Hz, 1H), 4.25 (s, 1H), 3.26 - 3.23I (m, 2H), 1.71 - 1.67 (m, 2H), 1.50 - 1.44 (m, 2H), 0.98 (t, J = 7.2 Hz, 3H) |
| 22 | 8-Bromo-N-isobutylquino xalin-6-amine | 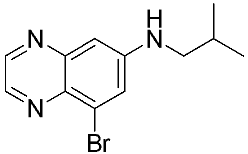 | 1H NMR (600 MHz, CDCl3) δ 8.63 (d, J = 1.8 Hz, 1H), 8.62 (d, J = 1.8 Hz, 1H), 7.53 (d, J = 3.0 Hz, 1H), 6.98 (d, J =2.4 Hz, 1H), 4.47 (s, 1H), 3.10 (d, J = 6.6 Hz, 2H), 2.04 - 1.99 (m, 1H), 1.04 (d, J = 6.6 Hz, 6H) |

### Preparation of Example Compounds:

### <Example 1> Preparation of 8-(6-(3-(3-fluorophenoxy)azetidin-1-yl)pyridin-3-yl)quinoxalin-6-amine

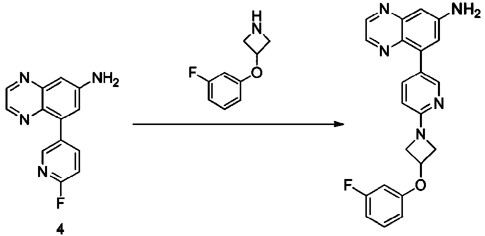

**A mixture of Intermediate Compound 4 and 3-(3-fluorophenoxy)azetidine hydrochloride (1.5 eq.)** was placed in DMF. To this mixture was added K2CO3 (5 eq.), and the mixture was stirred in a sealed tube at 100°C for 24-30 hours. The reaction was monitored by TLC (eluent = n-hexane/ethyl acetate: 4/6). After completion of the reaction, water was added to the reaction mixture and extracted with ethyl acetate. The organic layer was washed with cold water, dried over sodium sulfate, and evaporated under reduced pressure to give a crude material. The crude material was purified by silica gel column chromatography eluting with ethyl acetate/hexane to give 8-(6-(3-(3-fluorophenoxy)azetidin-1-yl)pyridin-3-yl)quinoxalin-6-amine.

Yellow solid; 75% yield; ¹H NMR (600 MHz, CDCl3) δ 8.65 (d, J = 1.8 Hz, 1H), 8.55 (d, J = 1.8 Hz, 1H), 8.44 (dd, J = 2.2 Hz, 1H), 7.86 (dd, J = 2.4 Hz, 1H), 7.26-7.23 (m, 1H), 7.17 (d, J = 2.6 Hz, 1H), 7.13 (d, *J* = 2.6 Hz, 1H), 6.73-6.70 (m, 1H), 6.59 (dd, J = 1.8Hz, 1H), 6.54 (dt, *J* = 2.4 Hz, 1H), 6.47 (dd, J = 8.4 Hz, 1H), 5.11 - 5.07 (m, 1H), 4.52 - 4.50 (m, 2H), 4.33 (s, 2H), 4.16 - 4.14 (m, 2H). ¹³C NMR (150 MHz, CDCl₃) δ 164.49, 162.87, 159.68, 158.02, 149.08, 147.72, 145.50, 144.99, 140.57, 139.58, 139.07, 136.28, 130.61, 123.53, 121.27, 110.32, 108.49, 107.56, 105.29, 102.69, 67.07, 57.76.

### <Example 2> Preparation of 8-(6-(3-(3-fluorophenoxy)azetidin-1-yl)pyridin-3-yl)-N-methylquinoxalin-6-amine

The target compound was obtained in a similar manner to Example 1, except that Intermediate Compound 5 was used.

Yellow solid; 43% yield; ¹H NMR (600 MHz, CDCl3) δ 8.64 (s, 1H), 8.51 (s, 1H), 8.42 (s, 1H), 8.08 (s, 1H), 7.87 - 7.86 (m, 1H), 7.10 (s, 1H), 6.95 (s, 1H), 6.72 (d, *J* = 7.2 Hz, 1H), 6.60 (d, *J* = 6.0 Hz, 1H), 6.53 (s, 1H), 6.49 - 6.46 (m, 1H), 5.09 (broad s, 1H), 5.01 (broad s, 1H), 4.52 - 4.50 (m, 2H), 4.42 (d, *J* = 4.2 Hz, 1H), 4.16 - 4.14 (m, 1H), 3.01 (s, 3H).

### <Example 3> Preparation of N-ethyl-8-(6-(3-(3-fluorophenoxy)azetidin-1-yl)pyridin-3-yl)quinoxalin-6-amine

The target compound was obtained in a similar manner to the preparation method of Example 1, except that Intermediate Compound 6 was used.

Yellow solid; 39% yield; 1H NMR (600 MHz, CDCl3) δ 8.62 (d, J = 1.2 Hz, 1H), 8.51 (d, *J =1.2* Hz, 1H), 8.45 (d, *J* = 2.4 Hz, 1H), 8.08 (s, 1H), 7.85 (d, *J* = 8.4 Hz, 1H), 7.10 (d, J = 2.4 Hz, 1H), 6.94 (d, J = 2.4 Hz, 1H), 6.75 - 6.72 (m, 2H), 6.65 (d, *J* = 8.4 Hz, 1H), 6.54 - 6.52 (m, 1H), 6.50 - 6.46 (m, 1H), 5.04 - 5.00 (m, 1H), 4.56 - 4.52 (m, 1H), 4.44 - 4.41 (m, 1H), 4.22 - 4.20 (m, 1H), 4.09 - 4.07 (m, 1H), 3.37 - 3.34 (m, 2H), 1.37 (t, J = 7.2 Hz, 3H). ESI-MS *m*/*z* [M + H]⁺ Anal. Calcd. for C₂₄H₂₂FN₅O 415.18, found 416.28.

### <Example 4> Preparation of 8-(6-(3-(3-fluorophenoxy)azetidin-1-yl)pyridin-3-yl)-N-propylquinoxalin-6-amine

The target compound was obtained in a similar manner to the preparation method of Example 1 using Intermediate Compound 7.

Yellow solid; 27% yield; ¹H NMR (600 MHz, CDCl₃) δ 8.65 (d, *J* = 1.8 Hz, 1H), 8.51 (d, *J* = 1.8 Hz, 1H), 8.45 (d, *J* = 1.8 Hz, 1H), 7.88 (dd, *J* = 2.4 Hz, 1H), 7.27 - 7.26 (m, 1H), 7.11 (d, *J* = 2.4 Hz, 1H), 6.98 (d, *J* = 2.4 Hz, 1H), 6.75 - 6.72 (m, 1H), 6.62 (dd, *J* = 2.4 Hz, 1H), 6.56 (dt, *J* = 2.4 Hz, 1H), 6.49 (d, *J* = 8.6 Hz, 1H), 5.14 - 5.10 (m, 1H), 4.55 - 4.52 (m, 2H), 4.33 (t, *J* = 5.4 Hz, 1H), 4.19 - 4.17 (m, 2H), 3.29 (q, *J =* 7.2, 5.4 Hz, 2H), 1.81 - 1.75 (m, 2H), 1.08 (t, *J* = 7.2 Hz, 3H).

### <Example 5> Preparation of 8-(6-(3-(3-fluorophenoxy)azetidin-1-yl)pyridin-3-yl)-N-isopropylquinoxalin-6-amine

The target compound was obtained in a similar manner to the preparation method of Example 1, except that Intermediate Compound 8 was used.

Yellow solid; 35% yield; ¹H NMR (600 MHz, CDCl₃) is 8.62 (d, *J* = 1.8 Hz, 1H), 8.49 (d, *J* = 1.8 Hz, 1H), 8.43 (d, *J* = 1. 8 Hz, 1H), 8.09 (s, 1H), 7.86 (dd, *J =* 2.4 Hz, 1H), 7.25 - 7.24 (m, 1H), 7.06 (d, *J* = 3.0 Hz, 1H), 6.95 (d, *J* = 2.4 Hz, 1H), 6.75 - 6.70 (m, 1H), 6.61 - 6.59 (m, 1H), 6.47 (d, *J* = 8.6 Hz, 1H), 5.12 - 5.08 (m, 1H), 5.03 - 5.00 (m, 1H), 4.56 - 4.51 (m, 3H), 4.17 - 4.15 (m, 2H), 1.33 (d, *J* = 6.6 Hz, 6H).

### <Example 6> Preparation of N-butyl-8-(6-(3-(3-fluorophenoxy)azetidin-1-yl)pyridin-3-yl)quinoxalin-6-amine

The target compound was obtained in a similar manner to the preparation method of Example 1, except that Intermediate Compound 9 was used.

Yellow solid; 27% yield; ¹H NMR (400 MHz, CDCl₃) is 8.62 (d, *J* = 1.6 Hz, 1H), 8.49 (d, *J* = 1.6 Hz, 1H), 8.43 (d, *J* = 1.6 Hz, 1H), 7.86 (dd, *J* = 2.4 Hz, 1H), 7.28 - 7.23 (m, 1H), 7.09 (d, *J* = 2.8 Hz, 1H), 6.96 (d, *J* = 2.4 Hz, 1H), 6.74 - 6.69 (m, 1H), 6.61 - 6.59 (m, 1H), 6.54 (dt, *J* = 2.4 Hz, 1H), 6.47 (d, *J* = 8.4 Hz, 1H), 5.13 - 5.07 (m, 1H), 4.54 - 4.50 (m, 2H), 4.27 (broad s, 1H), 4.17 - 4.14 (m, 2H), 3.32 - 3.27 (m, 2H), 1.75-1.68 (m, 2H), 1.54-1.44 (m, 2H), 0.99 (t, *J* = 7.2 Hz, 3H).

### <Example 7> Preparation of 8-(6-(3-(3-fluorophenoxy)azetidin-1-yl)pyridin-3-yl)-N-isobutylquinoxalin-6-amine

The target compound was obtained in a similar manner to the preparation method of Example 1, except that Intermediate Compound 10 was used.

Yellow solid; 31% yield; ¹H NMR (600 MHz, CDCl₃) δ 8.62 (d, *J* = 1.9 Hz, 1H), 8.49 (d, *J* = 1.9 Hz, 1H), 8.44 - 8.42 (m, 1H), 7.87 (dd, *J* = 2.4 Hz, 1H), 7.28 - 7.24 (m, 1H), 7.11 (d, *J* = 3.0 Hz, 1H), 6.95 (d, *J* = 2.4 Hz, 1H), 6.74-6.71 (m, 1H), 6.60 (dd, *J* = 1.8 Hz, 1H), 6.55 (dt, *J* = 2.4 Hz, 1H), 6.48-6.47 (m, 1H), 5.12 - 5.09 (m, 1H), 4.54 - 4.51 (m, 2H), 4.37 (broad s, 1H), 4.18 - 4.15 (m, 2H), 3.12 (t, *J* = 6.0 Hz, 2H), 2.07-2.00 (m, 1H), 1.05 (d, *J* = 6.4 Hz, 6H).

### <Example 8> Preparation of N-(8-(6-(3-(3-fluorophenoxy)azetidin-1-yl)pyridin-3-yl)quinoxalin-6-yl)furan-2-carboxamide

The target compound was obtained in a similar manner to the preparation method of Example 1, except that Intermediate Compound 11 was used.

Yellow solid; 21% yield; ¹H NMR (600 MHz, CDCl₃) δ 8.84 (d, *J* = 1.4 Hz, 1H), 8.77 (d, *J* = 1.4 Hz, 1H), 8.53 (d, *J* = 1.8 Hz, 1H), 8.45 - 8.43 (m, 2H), 8.07 (d, *J* = 2.4 Hz, 1H), 7.91 (dd, *J* = 2.4 Hz, 1H), 7.58 (s, 1H), 7.34 (d, *J* = 3.0 Hz, 1H), 7.28- 7.25 (m, 1H), 6.74 - 6.71 (m, 1H), 6.62 - 6.60 (m, 2H), 6.56 - 6.54 (m, 1H), 6.49 (d, *J* = 8.4 Hz, 1H), 5.13 - 5.10 (m, 1H), 4.55 - 4.53 (m, 2H), 4.19 - 4.17 (m, 2H). ¹³C NMR (150 MHz, CDCl₃) δ = 164.51, 162.89, 159.68, 156.23, 149.50, 147.38, 145.45, 144.63, 144.32, 143.32, 139.57, 139.15, 138.50, 130.62, 123.09, 123.00, 116.46, 116.16, 112.94, 110.32, 108.52, 108.38, 105.38, 102.71, 102.55, 67.07, 57.75.

### <Example 9> Preparation of N-(8-(6-(3-(3-fluorophenoxy)azetidin-1-yl)pyridin-3-yl)quinoxalin-6-yl)picolinamide

The target compound was obtained in a similar manner to the preparation method of Example 1, except that Intermediate Compound 12 was used.

Yellow solid; 24% yield; ¹H NMR (600 MHz, CDCl₃) is 10.42 (s, 1H), 8.85 (d, *J* = 1.8 Hz, 1H) , 8.77 (d, *J* = 1.8 Hz, 1H), 8.69 - 8.67 (m, 1H), 8.61 (d, *J* = 2.4 Hz, 1H), 8.56 - 8.55 (m, 1H), 8.37 (d, *J* = 7.8 Hz, 1H), 8.17 (d, *J* = 2.4 Hz, 1H), 7.98 - 7.95 (m, 1H), 7.93 (dd, J = 2.4 Hz, 1H) , 7.56-7.54 (m, 1H), 7.29 - 7.27 (m, 1H), 6.74-6.71 (m, 1H), 6.62 - 6.61 (m, 1H), 6.56 (dt, *J* = 2.4 Hz, 1H), 6.50 (d, *J* = 9.6 Hz, 1H), 5.14 - 5.11 (m, 1H), 4.56 - 4.53 (m, 2H), 4.20 - 4.18 (m, 2H).

### <Example 10> Preparation of (S)-N-(1-(5-(7-aminoquinoxalin-5-yl)pyridin-2-yl)pyrrolidin-3-yl)-2-fluorobenzamide

The target compound was obtained in a similar manner to the preparation method of Example 1, except that (S)-2-fluoro-N-(pyrrolidin-3-yl)benzamide was used instead of 3-(3-fluorophenoxy)azetidine hydrochloride.

Yellow solid; 72% yield; ¹H NMR (600 MHz, CDCl₃) is 8.65 (d, *J* = 1.8 Hz, 1H), 8.56 (d, *J* = 1.8 Hz, 1H), 8.45 (d, *J* = 2.4 Hz, 1H), 8.12 - 8.09 (m, 2H), 7.87 (dd, *J* = 9.0, 2.4 Hz, 1H), 7.50 - 7.48 (m, 1H),7.29 - 7.27 (m, 1H), 7.19 (d, *J* = 2.4 Hz, 1H), 7.14 - 7.13 (m, 1H), 6.93 - 6.90 (m, 1H), 6.54 (d, *J* = 8.4 Hz, 1H), 4.88 (bs, 1H), 4.26 (s, 2H), 3.95 - 3.91 (m, 1H), 3.74 - 3.69 (m, 2H), 3.60 - 3.58 (m, 1H), 2.49 - 2.43 (m, 1H), 2.37 - 2.33 (m, 1H).

### <Example 11> Preparation of (S)-2-fluoro-N-(1-(5-(7-(methylamino)quinoxalin-5-yl)pyridin-2-yl)pyrrolidin-3-yl)benzamide

The target compound was obtained in a similar manner to the preparation method of Example 2, except that (S)-2-fluoro-N-(pyrrolidin-3-yl)benzamide was used instead of 3-(3-fluorophenoxy)azetidine hydrochloride.

Yellow solid; 25% yield; ¹H NMR (400 MHz, CDCl₃) δ 8.63 (d, *J* = 2.0 Hz, 1H), 8.51 (d, *J* = 2.0 Hz, 1H), 8.45 (d, *J* = 2.4 Hz, 1H), 8.13-8.09 (m, 1H), 7.87 (dd, *J* = 8.8, 2.4 Hz, 1H), 7.51-7.45 (m, 1H), 7.30-7.28 (m, 1H), 7.12 - 7.10 (m, 2H), 6.95 (d, *J* = 2.4 Hz, 1H), 6.93 - 6.88 (m, 1H), 6.53 (d, *J* = 8.8 Hz, 1H), 4.91 - 4.85 (m, 1H), 4.35 (d, *J* = 5.2 Hz, 1H), 3.95 - 3.91 (m, 1H), 3.74 - 3.69 (m, 2H), 3.60 - 3.59 (m, 1H), 3.02 (d, *J* = 4.8 Hz, 3H), 2.50-2.41 (m, 1H), 2.22 - 2.13 (m, 1H).

### <Example 12> Preparation of (S)-N-(1-(5-(7-(ethylamino)quinoxalin-5-yl)pyridin-2-yl)pyrrolidin-3-yl)-2-fluorobenzamide

The target compound was obtained in a similar manner to the preparation method of Example 3, except that (S)-2-fluoro-N-(pyrrolidin-3-yl)benzamide was used instead of 3-(3-fluorophenoxy)azetidine hydrochloride.

Yellow solid; 27% yield; ¹H NMR (600 MHz, CDCl₃) δ 8.62 (d, *J* = 1.8 Hz, 1H), 8.50 (d, *J* = 1.8 Hz, 1H), 8.45 (d, *J* = 2.0 Hz, 1H), 8.12 - 8.09 (m, 1H), 7.86 (dd, *J* = 8.7 Hz, 1H), 7.50 - 7.46 (m, 1H), 7.29 - 7.27 (m, 1H), 7.14 - 7.10 (m, 2H), 6.95 (d, *J* = 2.5 Hz, 1H), 6.93 - 6.90 (m, 1H), 6.53 (d, *J* = 8.7 Hz, 1H), 4.88 (bs, 1H), 4.23 (s, 1H), 3.94 - 3.91 (m, 1H), 3.74 - 3.67 (m, 2H), 3.60 - 3.57 (m, 1H), 3.35-3.34 (m, 2H), 2.48 - 2.43 (m, 1H), 2.19 - 2.14 (m, 1H), 1.37 (t, *J* = 7.2 Hz, 3H).

### <Example 13> Preparation of (S)-2-fluoro-N-(1-(5-(7-(propylamino)quinoxalin-5-yl)pyridin-2-yl)pyrrolidin-3-yl)benzamide

The target compound was obtained in a similar manner to the preparation method of Example 4, except that (S)-2-fluoro-N-(pyrrolidin-3-yl)benzamide was used instead of 3-(3-fluorophenoxy)azetidine hydrochloride.

Yellow solid; 38% yield; ¹H NMR (600 MHz, CDCl₃) δ 8.62 (d, *J* = 2.0 Hz, 1H), 8.49 (d, *J* = 2.0 Hz, 1H), 8.45 (d, *J* = 2.0 Hz, 1H), 8.13 - 8.09 (m, 1H), 7.86 (dd, *J* = 8.4, 2.4 Hz, 1H), 7.51 - 7.45 (m, 1H), 7.29 (d, *J* = 7.2 Hz, 1H), 7.14 - 7.10 (m, 1H), 6.95 (d, *J =* 2.4 Hz, 1H), 6.95 (d, *J* = 2.4 Hz, 1H), 6.93 - 6.88 (m, 1H), 6.53 (d, *J* = 8.4 Hz, 1H), 4.88 (broad d, *J* = 4.8 Hz, 1H), 4.29 (bs, 1H), 3.95 - 3.91 (m, 1H), 3.74 - 3.68 (m, 2H), 3.60 - 3.57 (m, 1H), 3.27 (q, *J* = 5.2 Hz, 2H), 2.50 - 2.41 (m, 1H), 2.20 - 2.13 (m, 1H), 1.80 - 1.70 (m, 2H), 1.33 (t, *J* = 7.2 Hz, 3H).

### <Example 14> Preparation of (S)-2-fluoro-N-(1-(5-(7-(isopropylamino)quinoxalin-5-yl)pyridin-2-yl)pyrrolidin-3-yl)benzamide

The target compound was obtained in a similar manner to the preparation method of Example 5, except that (S)-2-fluoro-N-(pyrrolidin-3-yl)benzamide was used instead of 3-(3-fluorophenoxy)azetidine hydrochloride.

Yellow solid; 60% yield; ¹H NMR (600 MHz, CDCl₃) is 8.61 (d, *J* = 1.6 Hz, 1H), 8.49 (d, *J* = 1.6 Hz, 1H), 8.45 (d, *J* = 1.8 Hz, 1H), 8.13 - 8.10 (m, 1H), 7.86 (dd, *J* = 9.0, 2.4 Hz, 1H), 7.50 - 7.47 (m, 1H), 7.29 (d, *J* = 7.2 Hz, 1H), 7.14 - 7.11 (m, 1H), 7.07 (d, *J* = 2.4 Hz, 1H), 6.94 (d, *J* = 2.4 Hz, 1H), 6.92 - 6.89 (m, 1H), 6.53 (d, *J* = 9.0 Hz, 1H), 4.88 (broad d, *J* = 4.8 Hz, 1H), 4.14 - 4.11 (m, 1H), 3.94 - 3.92 (m, 1H), 3.83 (bs, 1H), 3.74 - 3.67 (m, 2H), 3.60 - 3.58 (m, 1H), 2.47 - 2.43 (m, 1H), 2.19 - 2.14 (m, 1H), 1.33 (d, *J* = 6.6 Hz, 6H).

### <Example 15> Preparation of (S)-N-(1-(5-(7-(butylamino)quinoxalin-5-yl)pyridin-2-yl)pyrrolidin-3-yl)-2-fluorobenzamide

The target compound was obtained in a similar manner to the preparation method of Example 6, except that (S)-2-fluoro-N-(pyrrolidin-3-yl)benzamide was used instead of 3-(3-fluorophenoxy)azetidine hydrochloride.

Yellow solid; 32% yield; ¹H NMR (600 MHz, CDCl₃) is 8.61 (d, *J* = 1.8 Hz, 1H), 8.49 (d, *J* = 1.8 Hz, 1H), 8.45 (d, *J* = 2.0 Hz, 1H), 8.12-8.09 (m, 1H), 7.86 (dd, *J* = 9.0, 2.4 Hz, 1H), 7.50 - 7.46 (m, 1H), 7.29 - 7.27 (m, 1H), 7.13 - 7.11 (m, 1H), 7.10 (d, *J* = 2.4 Hz, 1H), 6.94 (d, *J* = 2.4 Hz, 1H), 6.93 - 6.90 (m, 1H), 6.52 (d, *J* = 9.0 Hz, 1H), 4.88 (broad d, *J* = 4.8 Hz, 1H), 4.30 (bs, 1H), 3.94 - 3.91 (m, 1H), 3.73 - 3.68 (m, 2H), 3.59 - 3.57 (m, 1H), 3.29 (q, *J* = 6.6 Hz, 2H), 2.48 - 2.42 (m, 1H), 2.19 - 2.14 (m, 1H), 1.74-1.69 (m, 2H), 1.52 - 1.46 (m, 2H), 0.99 (t, *J* = 7.2 Hz, 3H).

### <Example 16> Preparation of (S)-2-fluoro-N-(1-(5-(7-(isobutylamino)quinoxalin-5-yl)pyridin-2-yl)pyrrolidin-3-yl)benzamide

The target compound was obtained in a similar manner to the preparation method of Example 7, except that (S)-2-fluoro-N-(pyrrolidin-3-yl)benzamide was used instead of 3-(3-fluorophenoxy)azetidine hydrochloride.

Yellow solid; 20% yield; ¹H NMR (600 MHz, CDCl₃) δ 8.62 (s, 1H), 8.50 (s, 1H), 8.46 (s, 1H), 8.13 - 8.10 (m, 1H), 7.87 (d, *J* = 8.4 Hz, 1H), 7.51 - 7.48 (m, 1H), 7.30 - 7.28 (m, 1H), 7.13 - 7.11 (m, 2H), 6.94 (d, *J =* 1.6 Hz, 1H), 6.92 - 6.89 (m, 1H), 6.54 (d, *J* = 8.6 Hz, 1H), 4.89 (broad d, *J* = 4.6 Hz, 1H), 4.36 (bs, 1H), 3.95 - 3.92 (m, 1H), 3.74-3.70 (m, 2H), 3.60 - 3.58 (m, 1H), 3.13 (t, *J* = 6.6 Hz, 2H), 2.49 - 2.43 (m, 1H), 2.20 - 2.15 (m, 1H), 2.06 - 2.02 (m, 1H), 1.05 (d, *J* = 6.6 Hz, 6H).

### <Example 17> Preparation of (S)-N-(8-(6-(3-(2-fluorobenzamido)pyrrolidin-1-yl)pyridin-3-yl)quinoxalin-6-yl)furan-2-carboxamide

The target compound was obtained in a similar manner to the preparation method of Example 8, except that (S)-2-fluoro-N-(pyrrolidin-3-yl)benzamide was used instead of 3-(3-fluorophenoxy)azetidine hydrochloride.

Yellow solid; 20% yield; ¹H NMR (600 MHz, CDCl₃) is 8.82 (d, *J* = 1.8 Hz, 1H), 8.77 (d, *J* = 1.8 Hz, 1H), 8.55 (d, *J* = 1.8 Hz, 1H), 8.44 (d, *J* = 2.4 Hz, 1H), 8.27 (d, *J* = 14.4 Hz, 1H), 8.13 - 8.06 (m, 2H), 7.92 - 7.90 (m, 1H), 7.58 (s, 1H), 7.50 - 7.48 (m, 1H), 7.34 (d, *J* = 3.0 Hz, 1H), 7.28 (s, 1H), 7.14 - 7.11 (m, 1H), ), 6.94 - 6.91 (m, 1H), 6.62 - 6.61 (m, 1H), 6.55 (d, *J* = 8.4 Hz, 1H), 4.89 (s, 1H), 3.96 - 3.90 (m, 1H), 3.75 -3.69 (m, 2H), 3.64 - 3.59 (m, 1H), 2.50 - 2.44 (m, 1H), 2.37 - 2.33 (m, 1H).

### <Example 18> Preparation of (S)-N-(8-(6-(3-(2-fluorobenzamido)pyrrolidin-1-yl)pyridin-3-yl)quinoxalin-6-yl)picolinamide

The target compound was obtained in a similar manner to the preparation method of Example 9, except that (S)-2-fluoro-N-(pyrrolidin-3-yl)benzamide was used instead of 3-(3-fluorophenoxy)azetidine hydrochloride.

Yellow solid; 27% yield; ¹H NMR (600 MHz, CDCl₃) δ 10.41 (s, 1H), 8.84 (d, *J* = 1.2 Hz, 1H), 8.77 (d, *J* = 1.2 Hz, 1H), 8.68 (d, *J* = 4.2 Hz, 1H), 8.59 (dd, *J =* 12.0, 2.4 Hz, 2H), 8.36 (d, *J* = 7.8 Hz, 1H), 8.16 (d, *J* = 1.8 Hz, 1H), 8.13 - 8.10 (m, 1H), 7.98 - 7.91 (m, 2H), 7.56 - 7.53 (m, 1H), 7.51 - 7.46 (m, 1H), 7.29 (d, *J* = 7.8 Hz, 1H), 7.14 - 7.11 (m, 1H), 6.95 - 6.91 (m, 1H), 6.56 (d, *J* = 8.4 Hz, 1H), 4.90 (broad d, *J* = 4.8 Hz, 1H), 3.97 - 3.94 (m, 1H), 3.76 - 3.71 (m, 2H), 3.62 - 3.60 (m, 1H), 2.50-2.45 (m, 1H), 2.22 - 2.16 (m, 1H).

### <Example 19> Preparation of 8-(6-morpholinopyridin-3-yl)quinoxalin-6-amine

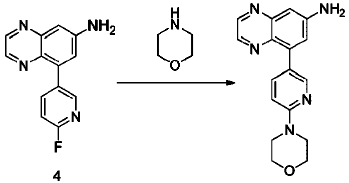

A mixture of Intermediate Compound 4 (1 eq.) and morpholine (1.5 eq.) was placed in DMSO, and K2CO3 (5 eq.) was added, followed by microwave heating of the reaction mixture at 120°C for 3 hours using an Anton Paar Microwave 300. The reaction was monitored by TLC (eluent = n-hexane/ethyl acetate: 4/6). After completion of the reaction, water was added to the reaction mixture and extracted with ethyl acetate. The organic layer was dried over sodium sulfate and evaporated under reduced pressure to give a crude material. The crude material was purified by silica gel column chromatography eluting with ethyl acetate/hexane to give 8-(6-morpholinopyridin-3-yl)quinoxalin-6-amine (yellow solid).

Yellow solid; 40% yield; ¹H NMR (600 MHz, CDCl₃) δ 8.67 (d, *J* = 1.7 Hz, 1H), 8.57 (d, *J* = 1.7 Hz, 1H), 8.49 (d, *J* = 1.9 Hz, 1H), 7.90 (dd, *J* = 8.7, 2.2 Hz, 1H), 7.19 (d, *J* = 2.3 Hz, 1H), 7.15 (d, *J* = 2.3 Hz, 1H), 6.77 (d, *J* = 8.7 Hz, 1H), 4.25 (s, 2H), 3.87 - 3.84 (m, 4H), 3.61 - 3.58 (m, 4H).

### <Example 20> Preparation of 8-(6-(4-methylpiperazin-1-yl)pyridin-3-yl)quinoxalin-6-amine

The target compound was obtained in a similar manner to Example 19, except that a piperazine derivative was used instead of morpholine.

Yellow solid; 45% yield; ¹H NMR (600 MHz, CDCl₃) δ 8.65 (d, *J* = 1.8 Hz, 1H), 8.55 (d, *J* = 1.8 Hz, 1H), 8.47 (d, *J* = 3.0 Hz, 1H), 7.87 (d, *J* = 8.4 Hz, 1H), 7.18 (d, *J* = 3.0 Hz, 1H), 7.13 (d, *J* = 3.0 Hz, 1H), 6.77 (d, *J* = 8.4 Hz, 1H), 4.27 (s, 2H), 3.66 (t, *J* = 4.8 Hz, 4H), 2.55 (t, *J* = 4.8 Hz, 4H), 2.37 (s, 3H).

### <Example 21> Preparation of 8-(6-(4-ethylpiperazin-1-yl)pyridin-3-yl)quinoxalin-6-amine

The target compound was obtained in a similar manner to the preparation method of Example 20.

**Yellow solid; 45% yield; ¹H NMR (600 MHz, CDCl₃) δ 8.66 (d, *J* = 1.4 Hz,** 1H), 8.56 (d, *J* = 1.6 Hz, 1H), 8.48 (d, *J* = 1.7 Hz, 1H), 7.88 (dd, *J* = 8.7 Hz, 1H), 7.19 (d, *J* = 2.4 Hz, 1H), 7.13 (d, *J* = 2.3 Hz, 1H), 6.78 (d, *J* = 8.8 Hz, 1H), 4.25 (s, 2H), 3.67 (s, 4H), 2.60 (s, 4H), 2.52 - 2.47 (m, 2H), 1.16 (t, *J* = 7.2 Hz, 3H) .

### <Example 22> Preparation of 8-(6-(4-(ethylsulfonyl)piperazin-1-yl)pyridin-3-yl)quinoxalin-6-amine

The target compound was obtained in a similar manner to the preparation method of Example 20.

**Yellow solid; 24% yield; ¹H NMR (600 MHz, CDCl₃) δ 8.67 (d, *J* = 1.8 Hz,** 1H), 8.56 (d, *J* = 1.8 Hz, 1H), 8.48 (d, *J* = 1.8 Hz, 1H), 7.91 (dd, *J* = 8.7, 2.4 Hz, 1H), 7.19 (d, *J* = 3.0 Hz, 1H), 7.15 (d, *J* = 2.4 Hz, 1H), 6.79 (d, *J* = 9.0 Hz, 1H), 4.28 (s, 2H), 3.75 (t, 4.8 Hz, 4H), 3.43 (t, 4.8 Hz, 4H), 3.00 (q, *J* = 7.2 Hz, 2H), 1.41 (t, *J* = 7.2 Hz, 3H).

### <Example 23> Preparation of 8-(6-(4-(pyridin-2-yl)piperazin-1-yl)pyridin-3-yl)quinoxalin-6-amine

The target compound was obtained in a similar manner to the preparation method of Example 20.

**Yellow solid; 44% yield; ¹H NMR (600 MHz, CDCl₃) δ 8.67 (s, 1H), 8.57** - 8.56 (m, 1H), 8.50 (d, *J* = 0.8 Hz, 1H), 8.23 (d, *J* = 4.2 Hz, 1H), 7.92 - 7.88 (m, 1H), 7.53 (t, *J* = 7.8 Hz, 1H), 7.21 - 7.18 (m, 1H), 7.15 (d, *J* = 1.3 Hz, 1H), 6.82 (d, *J* = 8.8 Hz, 1H), 6.72 (d, *J* = 8.5 Hz, 1H), 6.68 - 6.66 (m, 1H), 4.25 (s, 2H), 3.81 - 3.77 (m, 4H), 3.74 - 3.71 (m, 4H).

### <Example 24> Preparation of 8-(6-(4-(4-bromobenzyl)piperazin-1-yl)pyridin-3-yl)quinoxalin-6-amine

The target compound was obtained in a similar manner to the preparation method of Example 20.

**Yellow solid; 35% yield; ¹H NMR (600 MHz, CDCl₃) δ 8.66 (d, *J* = 1.8 Hz,** 1H), 8.56 (d, *J* = 1.8 Hz, 1H), 8.47 (d, *J* = 2.4 Hz, 1H), 7.87 (dd, *J* = 8.4, 2.4 Hz, 1H), 7.48-7.42 (m, 3H), 7.25 (s, 1H), 7.18 (d, *J* = 2.4 Hz, 1H), 7.13 (d, *J* = 2.4 Hz, 1H), 6.75 (d, *J* = 9.0 Hz, 1H), 4.25 (s, 2H), 3.64 (t, 4.8 Hz, 4H), 3.52 (s, 2H), 3.43 (t, 4.8 Hz, 4H).

### <Example 25> Preparation of 8-(6-(4-morpholinopiperidin-1-yl)pyridin-3-yl)quinoxalin-6-amine

The target compound was obtained in a similar manner to Example 19, except that a piperidine derivative was used instead of morpholine.

Yellow solid; 56% yield; ¹H NMR (600 MHz, CDCl₃) δ 8.65 (d, *J* = 1.8 Hz, 1H), 8.56 (d, *J* = 1.8 Hz, 1H), 8.46 (d, *J* = 1.8 Hz, 1H), 7.86 (dd, *J* = 8.4, 3.0 Hz, 1H), 7.18 (d, *J* = 3.0 Hz, 1H), 7.13 (d, J = 2.4 Hz, 1H), 6.79 (d, *J* = 9.0 Hz, 1H), 4.44 (d, *J* = 13.2 Hz, 2H), 4.26 (s, 2H), 3.74 (t, *J* = 4.8 Hz, 4H), 2.94 - 2.89 (m, 2H), 2.94 - 2.89 (t, 4.8 Hz, 4H), 2.48 - 2.43 (m, 1H), 1.96 (d, *J* = 12.6 Hz, 2H), 1.60 - 1.54 (m, 2H) .

### <Example 26> Preparation of 8-(6-(4-(4-bromobenzyl)piperazin-1-yl)pyridin-3-yl)-N-ethylquinoxalin-6-amine

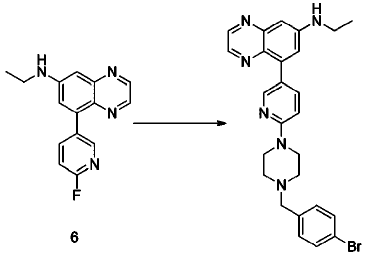

The target compound was obtained in a similar manner to Example 19, except that Intermediate Compound 6 was used as the reactant.

Yellow solid; 31% yield; ¹H NMR (600 MHz, CDCl₃) is 8.63 (d, *J* = 1.8 Hz, 1H), 8.50 (d, *J* = 1.8 Hz, 1H), 8.45 (d, *J* = 2.4 Hz, 1H), 7.86 (dd, *J* = 9.0, 2.4 Hz, 1H), 7.48 - 7.46 (m, 2H), 7.27 (s, 1H), 7.25 (s, 1H), 7.09 (d, *J* = 2.4 Hz, 1H), 6.96 (d, *J* = 2.4 Hz, 1H), 6.75 (d, *J* = 8.4 Hz, 1H), 4.21 (s, 1H), 3.63 (t, J = 4.8 Hz, 4H), 3.52 (s, 2H), 3.34 (q, J = 7.2 Hz, 2H), 2.57 (t, J = 4.8 Hz, 4H), 1.37 (t, J = 7.2 Hz, 3H).

### <Example 27> Preparation of 5-(6-(3-(3-fluorophenoxy)azetidin-1-yl)pyridin-3-yl)-7-nitroquinoxaline

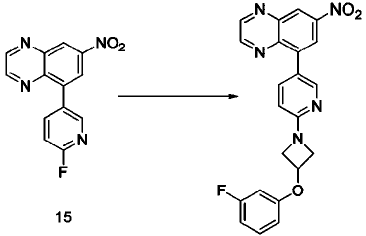

The target compound was obtained in a similar manner to Example 1, except that Intermediate Compound 15 was used.

Yellow solid; 17% yield; ¹H NMR (600 MHz, CDCl₃) δ 9.03 (d, *J* = 1.6 Hz, 1H), 9.00 (d, *J* = 1.7 Hz, 1H), 8.96 (d, *J* = 2.5 Hz, 1H), 8.57 - 8.54 (m, 2H), 7.91 (dd, *J* = 8.6 Hz, 2.4, 1H), 7.29 - 7.25 (m, 1H), 6.75 - 6.71 (m, 1H), 6.61 (dd, *J* = 8.2 Hz, 1H), 6.55 (dt, *J =* 10.5 Hz, 1H), 6.51 (dd, *J* = 8.6 Hz, 1H), 5.13 (m, 1H), 4.56 (m, 2H), 4.22 - 4.18 (m, 2H).

### <Example 28> Preparation of N,N-diethyl-8-(6-(3-(3-fluorophenoxy)azetidin-1-yl)pyridin-3-yl)quinoxalin-6-amine

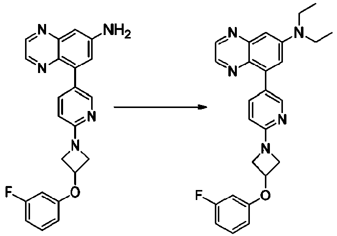

To a solution of the compound prepared in Example 1 (1 eq.) dissolved in DMF was added DIPEA (2 eq.), followed by the addition of ethyl iodide (2.0 eq.) at room temperature. The reaction mixture was then stirred at 80°C for 16 hours and monitored by TLC (eluent = n-hexane/ethyl acetate: 6/4). After completion of the reaction, water was added to the reaction mixture and extracted with ethyl acetate. The organic layer was washed with brine, dried over sodium sulfate, and evaporated under reduced pressure to give a crude material. The crude material was purified by silica gel column chromatography eluting with ethyl acetate/hexane to give N,N-diethyl-8-(6-(3-(3-fluorophenoxy)azetidin-1-yl)pyridin-3-yl)quinoxalin-6-amine.

Yellow solid; 21% yield; ¹H NMR (600 MHz, CDCl₃) δ 8.60 (d, *J* = 1.9 Hz, 1H), 8.46 (d, *J* = 1.9 Hz, 1H), 8.45 (d, *J* = 2.0 Hz, 1H), 7.91 (dd, *J* = 8.5 Hz, 1H), 7.43 (s, 1H), 7.29 (d, *J* = 2.9 Hz, 1H), 7.04 (d, *J* = 2.9 Hz, 1H), 6.73 - 6.70 (m, 1H), 6.60 (dd, *J* = 8.4 Hz, 1H), 6.56 - 6.53 (m, 1H), 6.48 (d, *J* = 8.6 Hz, 1H), 5.11 (m, 1H), 4.54 - 4.50 (m, 2H), 4.17 - 4.15 (m, 2H), 3.56 - 3.53 (q, *J* = 14.2 Hz, 4H), 1.25 (bs, 6H).

### <Example 29> Preparation of 8-(2-(3-(3-fluorophenoxy)azetidin-1-yl)pyrimidin-5-yl)quinoxalin-6-amine

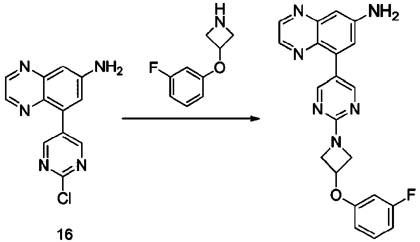

The target compound was obtained in a similar manner to Example 1, except that Intermediate Compound 16 was used.

Yellow solid; 50% yield; ¹H NMR (600 MHz, CDCl₃) δ 8.69 (s, 2H), 8.68 (d, *J* = 2.4 Hz, 1H), 8.55 (d, *J* = 3.0 Hz, 1H), 7.29 - 7.27 (m, 1H), 7.25 - 7.23 (m, 1H), 7.17 (q, *J* = 4.2 Hz, 1H), 6.75-6.70 (m, 1H), 6.61-6.58 (m, 1H), 6.56-6.53 (m, 1H), 5.11 - 5.06 (m, 1H), 4.65 - 4.60 (m, 2H), 4.31 - 4.27 (m, 4H).

### <Example 30> Preparation of (S)-N-(1-(5-(7-aminoquinoxalin-5-yl)pyrimidin-2-yl)pyrrolidin-3-yl)-2-fluorobenzamide

The target compound was obtained in a similar manner to Example 29.

**Yellow solid; 50% yield; ¹H NMR (600 MHz, CDCl₃) δ 8.70 (s, 2H), 8.68** (d, *J* = 1.8 Hz, 1H), 8.55 (d, *J* = 1.8 Hz, 1H), 8.13 - 8.10 (m, 1H), 7.47 - 7.50 (m, 1H), 7.30 - 7.28 (m, 1H), 7.18 (d, *J* = 2.4 Hz, 1H), 7.16 (d, *J* = 8.4 Hz, 1H), 7.14 - 7.11 (s, 1H), 6.91 - 6.88 (m, 1H), 4.97 - 4.87 (m, 1H), 4.28 (s, 2H), 4.07 - 4.04 (m, 1H), 3.84 (t, *J* = 6.6 Hz, 2H), 3.73 - 3.71 (m, 1H), 2.49 - 2.43 (m, 1H), 2.19 - 2.14 (m, 1H).

### <Example 31> Preparation of N-ethyl-8-(3-morpholinoprop-1-yn-1-yl)quinoxalin-6-amine

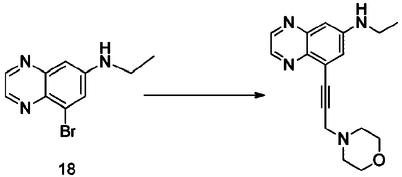

To **a solution of Intermediate Compound 18 (1 eq.), 4-(prop-2-yn-1-yl)morpholine (1.6 eq.), and TEA (2 eq.)** in 2% TPGS/H2O (0.3 mL) were added [Pd(Cinnamyl)Cl]2 (0.0005 eq.) and cBRIDP (0.003 eq.). The resulting reaction mixture was then stirred at 50°C for 1 hour. The reaction was monitored by TLC (eluent = DCM/MeOH: 9/1). After completion of the reaction, the reaction mixture was filtered through Celite. The filtrate was treated with water and extracted with 10% MeOH/DCM. The organic layer was dried over sodium sulfate and evaporated under reduced pressure to give a crude material. The crude material was purified by silica gel column chromatography eluting with 5% MeOH/DCM to give N-ethyl-8-(3-morpholinoprop-1-yn-1-yl)quinoxalin-6-amine.

Violet gummy; 23.5% yield; ¹H NMR (600 MHz, CDCl₃) δ 8.65 (s, 1H), 8.58 (s, 1H), 7.32 (s, 1H), 6.95 (s, 1H), 4.16 (s, 1H), 3.80 (s, 4H), 3.69 (s, 2H), 3.32 - 3.28 (m, 2H), 2.73 (s, 4H), 1.35 (t, *J* = 7.1 Hz, 3H). ¹³C NMR (150 MHz, CDCl₃) δ 148.50, 145.41, 145.27, 140.52, 137.95, 126.04, 123.55, 104.48, 90.19, 82.23, 66.92, 52.42, 48.39, 38.25, 14.40.

### <Example 32> Preparation of N7-ethyl-N5-(5-methyl-1H-pyrazol-3-yl)quinoxaline-5,7-diamine

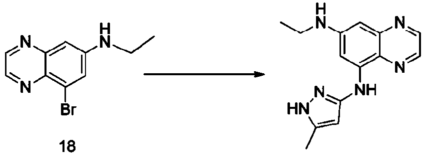

**To a degassed dry toluene solution of Intermediate Compound 18 (1 eq.), 5-methyl-1H-pyrazol-3-amine (1.3 eq.),** and Cs2CO3 (2.5 eq.) were added Pd2(dba)3 (0.1 eq.) and BINAP (0.2 eq.), and the mixture was stirred in a sealed tube at 100°C for 16 hours. The reaction was monitored by TLC (eluent = n-hexane/ethyl acetate: 2/8). After completion of the reaction, the reaction mixture was filtered through Celite. The filtrate was treated with water and extracted with ethyl acetate. The organic layer was dried over sodium sulfate and evaporated under reduced pressure to give a crude material. The crude material was purified by silica gel column chromatography eluting with ethyl acetate/hexane to give N7-ethyl-N5-(5-methyl-1H-pyrazol-3-yl)quinoxaline-5,7-diamine.

The structures of the compounds prepared in Examples 1-32 are summarized in Table 2 below.

**[Table 2]**

| **Example** | **Structure** | **Example** | **Structure** |
|---|---|---|---|
| 1 | 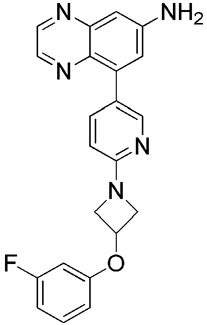 | 2 | 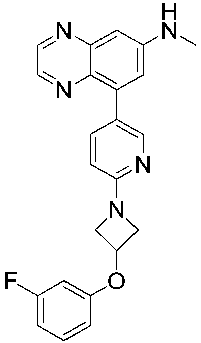 |
| 3 | 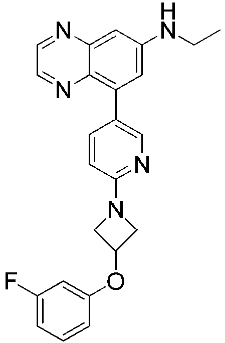 | 4 | 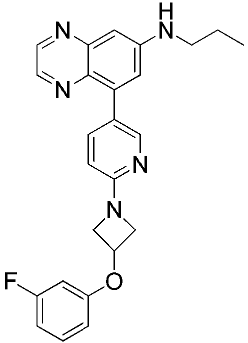 |
| 5 | 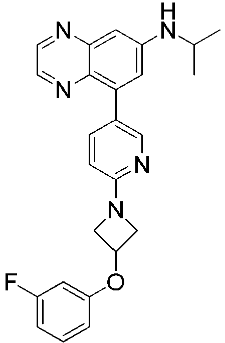 | 6 | 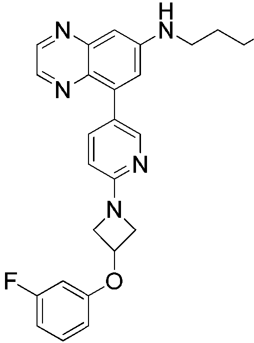 |
| 7 | 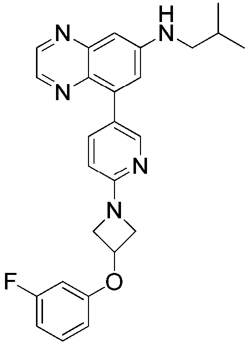 | 8 | 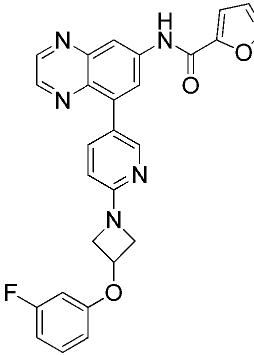 |
| 9 | 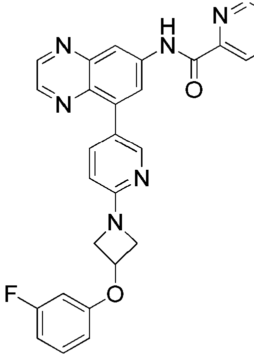 | 10 | 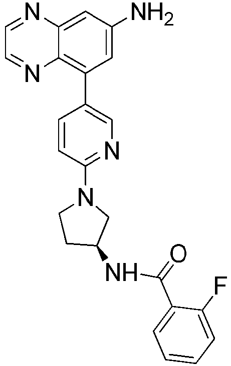 |
| 11 | 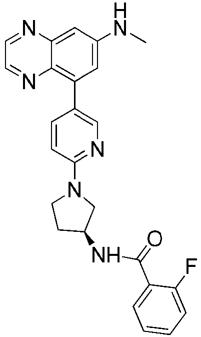 | 12 | 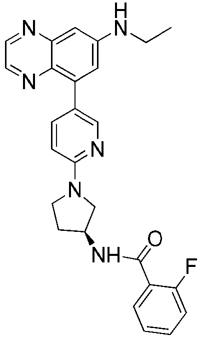 |
| 13 | 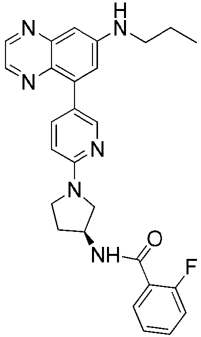 | 14 | 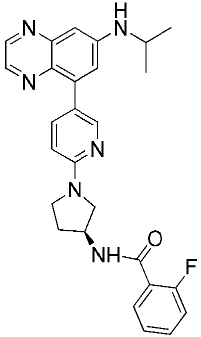 |
| 15 | 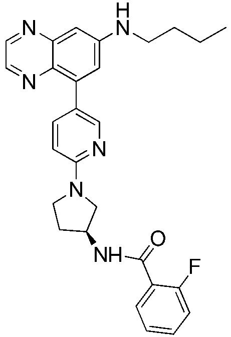 | 16 | 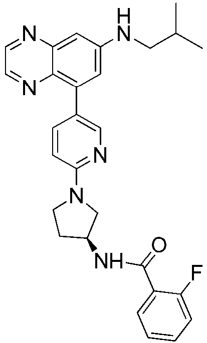 |
| 17 | 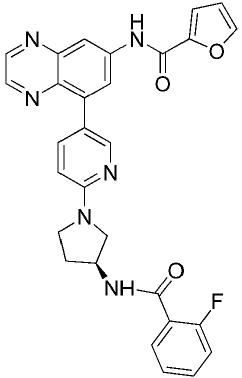 | 18 | 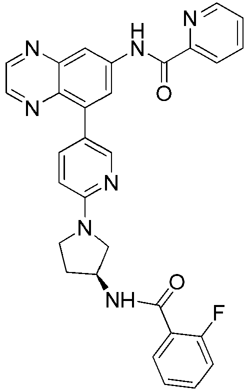 |
| 19 | 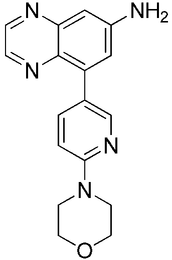 | 20 | 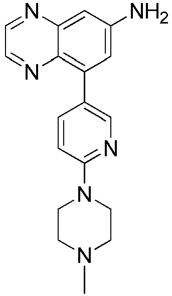 |
| 21 | 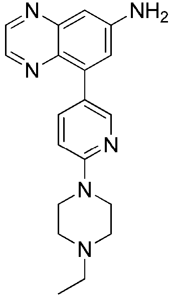 | 22 | 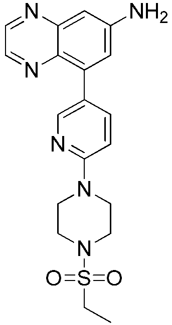 |
| 23 | 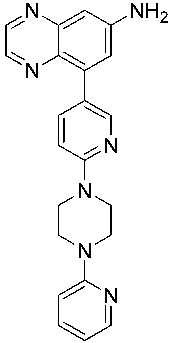 | 24 | 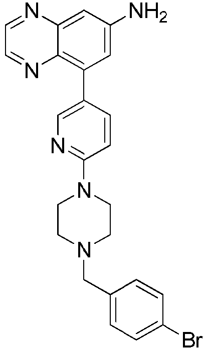 |
| 25 | 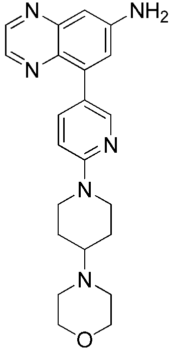 | 26 | 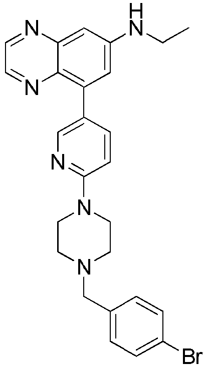 |
| 27 | 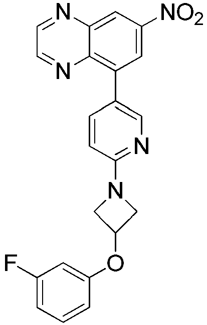 | 28 | 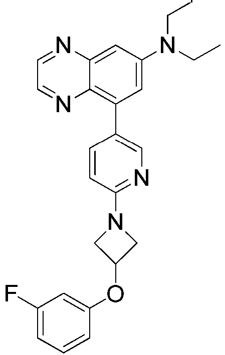 |
| 29 | 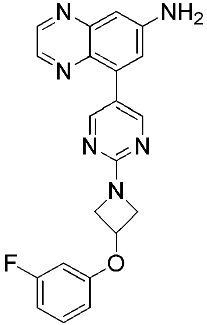 | 30 | 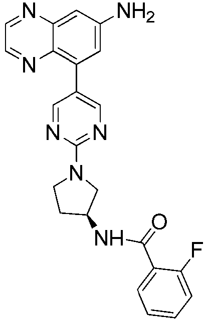 |
| 31 | 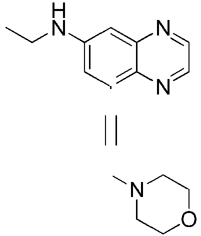 | 32 | 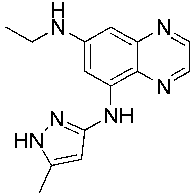 |

### <Experimental Example 1> Evaluation of Inhibitory Activity against Rearranged During Transfection (RET) Kinase

To evaluate the enzymatic inhibitory activity of the compounds represented by Formula I or Formula IV according to the present invention against RET kinase, the following experiment was performed. The results are shown in Tables 3 to 6 below.

### 1) Experimental Method

The inhibitory activities of the example and intermediate compounds of the present invention against a kinase panel (Reaction Biology Corp) of RET and RET mutations (RET(G810R), RET(V804M), KIF5B-RET, RET(V804M)-KIF5B, RET-CCDC6, RET-NCOA4, and RET(G810S/M918T)) were investigated. Using radiolabeled ATP ([γ-33P] ATP), the degree to which the substrate was replaced by a 33P-phosphorylated substrate was measured, and changes in kinase activity were determined from the measurement of the radiolabeled phosphorylated substrate. The example and intermediate compounds were tested at concentrations of 0.1 µM to 30 µM against ATP concentrations of 1 µM or 10 µM. The residual activity (%) of the example and intermediate compounds after inhibition against each kinase panel is shown in Tables 3 to 5 below. As a control compound (Control Cmpd), staurosporine was tested in a 10-dose IC50 mode with 4-fold serial dilutions starting from 20 µM to determine the IC50 values.

**[Table 3]**

| Example | % Enzyme Activity (relative to DMSO controls) at 1 uM & 0.1 uM | | | | | |
|---|---|---|---|---|---|---|
| | RET | G810R | V804M | RET (V804M )-KIF5B | RET-CCDC6 (PTC1) | RET-NCOA4 (PTC3) |
| 1 (at 1uM) | 0.51 | 24.47 | 1.72 | NT | 0.01 | 1.11 |
| 2 | NT | 77.27 | NT | NT | NT | NT |
| 3 (at 1uM) | 0.14 | 11.37 | 0.63 | NT | 18.57 | 16.83 |
| 8 | NT | 96.71 | 45.95 | 65.06 | NT | NT |
| 10 | NT | 59.9 | 2.27 | 5.50 | NT | NT |
| 11 | NT | 55.99 | NT | NT | NT | NT |
| 12 | NT | 59.90 | 2.27 | 5.50 | NT | NT |
| 27 (at 1uM) | 67.19 | 100.64 | 62.83 | NT | NT | NT |
| 28 (at 1uM) | 28.34 | 104.55 | 16.47 | NT | NT | NT |
| 29 | NT | 76.29 | NT | NT | NT | NT |
| 31 | NT | 98.48 | 104.89 | 109.38 | NT | NT |
| 32 | NT | 96.64 | 88.72 | 85.94 | NT | NT |
| Intermediate Compound 6 (at 1uM) | 57.03 | 97.94 | 59.34 | NT | NT | NT |
| Intermediate Compound 11 (at 1uM) | 93.74 | 97.56 | 87.11 | NT | NT | NT |
| Intermediate Compound 13 (at 1uM) | 95.74 | 101.57 | 104.14 | NT | NT | NT |
| Intermediate Compound 15 (at 1uM) | 93.54 | 99.45 | 94.89 | NT | NT | NT |
| Intermediate Compound 18 (at 1uM) | 71.08 | 104.01 | 68.33 | NT | NT | NT |
| IC50 (M) Control Cmpd | 3.98E-09 | 9.36E-07 | 1.83E-10 | 7.25E-10 | 3.60E-09 | 5.84E-09 |

| | | | | | | |
|---|---|---|---|---|---|---|
| * Example Compounds 1, 3, 27, and 28 and the intermediate compounds were tested at 1 µM, while the remaining compounds were tested at 0.1 µM. The assay was conducted at an ATP concentration of 10 µM. | | | | | | |

**[Table 4]**

| Example | | % Enzyme Activity (relative to DMSO controls) at 10 uM & 30 uM | | | |
|---|---|---|---|---|---|
| | | RET-CCDC6 (PTC1) | | | |
| | | | Data 1 | | Data 2 |
| | 1 | | 0.75 | | 0.52 |
| | 2 | | 1.38 | | 0.23 |
| | 3 | | 0.73 | | 0.91 |
| | 4 | | 1.11 | | 1.31 |
| | 5 | | 1.34 | | 0.72 |
| | 6 | | 1.05 | | 1.32 |
| | 7 | | 0.85 | | 1.07 |
| | 8 | | 2.53 | | 1.80 |
| | 9 | | 2.81 | | 3.74 |
| | 10 | | 1.55 | | 0.99 |
| | 11 | | 1.36 | | 1.07 |
| | 12 | | 1.25 | | 1.01 |
| | 13 | | 0.91 | | 1.28 |
| | 14 | | 1.04 | | 0.72 |
| | 15 | | 1.08 | | 1.10 |
| | 16 | | 1.21 | | 1.51 |
| | 17 | | 1.15 | | 1.33 |
| | 18 | | 1.90 | | 2.31 |
| | 19 | | 5.69 | | 6.45 |
| | 20 | | 12.94 | | 15.26 |
| | 21 | | 25.98 | | 27.99 |
| | 22 | | 2.90 | | 3.03 |
| | 23 | | 1.92 | | 2.29 |
| | 24 | | 1.51 | | 1.27 |
| | 25 | | 18.28 | | 20.41 |
| | 26 | | 0.94 | | 1.32 |
| | 29 | | 1.22 | | 1.07 |
| | 30 | | 1.19 | | 1.20 |
| | 31 | | 14.77 | | 15.41 |
| | 32 | | 5.39 | | 4.58 |
| Intermediate Compound 4 (at 30uM) | | | 20.90 | | 19.43 |
| Intermediate Compound 5 (at 30uM) | | | 5.67 | | 3.67 |
| Intermediate Compound 8 (at 30uM) | | | 7.38 | | 7.71 |
| Intermediate Compound 16 (at 30uM) | | | 13.24 | | 13.27 |
| Intermediate Compound 20 (at 30uM) | | | 7.86 | | 7.51 |
| IC50 (M) Control Cmpd | | | 1.39E-09 | | |

| | | | | | |
|---|---|---|---|---|---|
| * All intermediate compounds were tested at 30 µM, while the remaining compounds were tested at 10 µM. The assay was conducted at an ATP concentration of 1 µM. | | | | | |

**[Table 5]**

| | Example | % Enzyme Activity (relative to DMSO controls) | | | |
|---|---|---|---|---|---|
| | | | at 10 uM & 30 uM | | |
| | | | RET (G810S/M918T) | | |
| | | | Data 1 | | Data 2 |
| | 1 | | 0.22 | | -0.72 |
| | 2 | | 0.05 | | -0.43 |
| | 3 | | -0.47 | | -0.51 |
| | 4 | | -0.23 | | 0.04 |
| | 5 | | 5.96 | | 6.14 |
| | 6 | | 2.18 | | 2.37 |
| | 7 | | 4.28 | | 4.26 |
| | 8 | | 13.63 | | 12.93 |
| | 9 | | 49.52 | | 49.98 |
| | 10 | | -0.09 | | -0.60 |
| | 11 | | -0.84 | | -0.85 |
| | 12 | | 2.65 | | 0.69 |
| | 13 | | -0.06 | | -0.31 |
| | 14 | | 5.08 | | 4.68 |
| | 15 | | -0.06 | | -0.20 |
| | 16 | | 3.39 | | 3.22 |
| | 17 | | 11.90 | | 11.93 |
| | 18 | | 24.71 | | 25.52 |
| | 19 | | 27.31 | | 26.31 |
| | 20 | | 48.11 | | 45.04 |
| | 21 | | 66.71 | | 63.30 |
| | 22 | | 15.67 | | 15.09 |
| | 23 | | 9.23 | | 9.45 |
| | 24 | | 2.24 | | 2.38 |
| | 25 | | 46.76 | | 45.17 |
| | 26 | | 1.23 | | 1.30 |
| | 27 (at 30uM) | | 59.88 | | 58.14 |
| | 28 (at 30uM) | | 4.19 | | 4.35 |
| | 29 | | 0.28 | | 0.11 |
| | 30 | | 0.97 | | 0.98 |
| | 31 | | 61.67 | | 61.25 |
| | 32 | | 41.91 | | 40.62 |
| Intermediate Compound4 (at 30uM) | | | 53.51 | | 54.09 |
| Intermediate Compound 5 (at 30uM) | | | 13.29 | | 14.36 |
| Intermediate Compound 6 (at 30uM) | | | 18.24 | | 17.22 |
| Intermediate Compound 7 (at 30uM) | | | 24.02 | | 22.34 |
| Intermediate Compound 8 (at 30uM) | | | 66.54 | | 68.40 |
| Intermediate Compound 9 (at 30uM) | | | 24.80 | | 25.30 |
| Intermediate Compound 10 (at 30uM) | | | 43.15 | | 40.10 |
| Intermediate Compound 11 (at 30uM) | | | 74.71 | | 73.01 |
| Intermediate Compound 12 (at 30uM) | | | 100.18 | | 96.39 |
| Intermediate Compound 13 (at 30uM) | | | 92.56 | | 89.69 |
| Intermediate Compound 14 (at 30uM) | | | 92.51 | | 92.52 |
| Intermediate Compound 15 (at 30uM) | | | 97.38 | | 99.40 |
| Intermediate Compound 16 (at 30uM) | | | 40.77 | | 39.12 |
| Intermediate Compound 18 (at 30uM) | | | 10.29 | | 9.57 |
| Intermediate Compound 19 (at 30uM) | | | 27.55 | | 28.30 |
| Intermediate Compound 20 (at 30uM) | | | 69.07 | | 73.09 |
| Intermediate Compound 21 (at 30uM) | | | 20.81 | | 21.74 |
| Intermediate Compound 22 (at 30uM) | | | 49.44 | | 48.90 |
| IC50 (M) Control Cmpd | | | 3.36E-08 | | |

| | | | | | |
|---|---|---|---|---|---|
| * Example Compounds 27 and 28 and the intermediate compounds were tested at 30 µM, while the remaining compounds were tested at 10 µM. The assay was conducted at an ATP concentration of 1 µM. | | | | | |

Furthermore, the example compounds were tested in a 10-dose IC50 mode with 3-fold serial dilutions starting from 30 µM. Among the inhibitory activities of the example and intermediate compounds in Tables 3 to 5 for each kinase panel, examples showing significant results were selected and the IC50 values as indicators of inhibitory activity were determined and are shown in Table 6 below.

**[Table 6]**

| Example | | Enzymatic inhibitory activity (IC50) nM | | | | |
|---|---|---|---|---|---|---|
| | | RET | G810R | V804M | KIF5B-RET | KIF5B-RET (V804M) |
| | 1 | 3.90 | 275 | 4.2 | 2.2 | 6.71 |
| | 3 | 0.57 | 142 | 0.78 | <0.5 | 1.36 |
| | 10 | NT | 44.7 | 0.5 | NT | 2.26 |

As shown in Tables 3 to 6, it was confirmed that the example compounds exhibit inhibitory activity against RET kinase and various RET mutations including G810R, V804M, KIF5B-RET, RET(V804M)-KIF5B, RET-CCDC6, RET-NCOA4, and RET(G810S/M918T).

Therefore, as the example compounds can effectively inhibit the activity of RET kinase, they can be usefully used as a pharmaceutical composition for the prevention or treatment of RET kinase-related diseases, such as cancer, comprising the same as an active ingredient.

### <Experimental Example 2> Evaluation of Cell Proliferation Inhibition

To evaluate the inhibitory activity of the compounds represented by Formula I or Formula IV according to the present invention against RET mutation-driven cell growth in the Ba/F3 cell line and against cell growth in the non-small cell lung cancer cell line A-549, the following experiment was performed.

"Ba/F3" is an artificial rodent cell model that stably exhibits overexpression of RET mutations, and as a result, cellular activity does not necessarily correlate with RET enzyme activity.

### 1) Experimental Method

Each cell line was seeded in a 96-well plate at 1×10⁴ cells/90 µL/well, and 10 µL/well of culture medium containing each example and intermediate compound or DMSO control at 9 concentrations (1 nM to 100 µM) prepared by 3-fold serial dilutions was added to achieve a final concentration of 0 to 100 µM, followed by incubation for 72 hours in a 37°C, 5% CO2 incubator. After 72 hours of incubation, the compound-treated plates were removed, and Cell Counting Kit-8 solution was added at 10 µL/well and mixed well. The plates were then incubated for 2 hours in a CO2 incubator at 37°C, and the absorbance was measured at 450 nm using a microplate reader.

### 2) Results

### Inhibitory Activity against RET Mutation Cell Lines

The absorbance of the experimental group relative to that of the DMSO control group was converted to % survival, and the IC50 values representing the concentration required to inhibit cell growth by 50% were calculated. The results are shown in Table 7 below. As a control compound, selpercatinib, a known RET inhibitor, was treated to calculate the IC50 values.

**[Table 7]**

| Example | | Cell Viability of BaF3 Cell lines (IC50 in uM) | | | |
|---|---|---|---|---|---|
| | | KIF5B-RET (G810R) | | KIF5B-RET (V804M) | |
| | 1 | | 0.88 | | 1.02 |
| | 3 | | 1.09 | | 0.12 |
| | 27 | | >10 | | NT |
| | 28 | | >10 | | NT |
| Intermediate Compound 6 | | | 4.44 | | NT |
| Intermediate Compound 11 | | | >10 | | NT |
| Intermediate Compound 13 | | | >10 | | NT |
| Intermediate Compound 18 | | | 0.73 | | NT |
| Selpercatinib | | | 1.41 | | NT |

In addition, the data were expressed as a percentage relative to vehicle-treated cells, and GI50 values were calculated using GraphPad Prism 8.0 (GraphPad Software Inc., San Diego), and the results are shown in Table 8 below. As a control compound, ponatinib, a known RET inhibitor, was treated to calculate the GI50 values.

**[Table 8]**

| | Example | GI50 of BaF3-RET(V804M) | |
|---|---|---|---|
| | 1 | | 1.455 |
| | 2 | | 0.943 |
| | 3 | | 0.661 |
| | 11 | | 2.125 |
| | 12 | | 1.055 |
| | Ponatinib | | 0.115 |

### Inhibitory Activity against A-549 Cell Line

The data were expressed as a percentage relative to vehicle-treated cells, and GI50 values were calculated using GraphPad Prism 8.0 (GraphPad Software Inc., San Diego), and the results are shown in Table 9 below. As a control compound, ponatinib, a known RET inhibitor, was treated to calculate the GI50 values.

**[Tabel 9]**

| Example | Cell Proliferation (% of control) | SD |
|---|---|---|
| Ponatinib | -1.99 | 0.81 |
| 1 | 34.36 | 4.83 |
| 2 | 18.59 | 10.38 |
| 3 | 25.06 | 5.90 |
| 4 | 43.59 | 7.27 |
| 5 | 36.07 | 3.13 |
| 6 | 68.59 | 3.10 |
| 7 | 53.57 | 3.67 |
| 8 | 78.35 | 4.58 |
| 9 | 18.67 | 8.77 |
| 10 | 32.59 | 7.62 |
| 11 | 20.80 | 7.59 |
| 12 | 41.67 | 9.10 |
| 13 | 39.32 | 7.73 |
| 14 | 47.83 | 8.68 |
| 15 | 51.55 | 7.50 |
| 16 | 65.29 | 4.59 |
| 17 | 48.96 | 4.04 |
| 18 | 35.32 | 8.08 |
| 19 | 71.46 | 3.89 |
| 20 | 81.61 | 9.73 |
| 21 | 64.90 | 4.70 |
| 22 | 68.42 | 9.59 |
| 23 | 78.36 | 1.72 |
| 24 | 15.35 | 2.92 |
| 25 | 75.52 | 4.18 |
| 26 | 79.51 | 6.26 |
| 27 | 94.57 | 0.21 |
| 28 | 94.81 | 5.04 |
| 29 | 12.87 | 3.29 |
| 30 | 42.94 | 6.19 |
| 31 | 51.88 | 5.66 |
| 32 | 92.91 | 8.30 |

As shown in Tables 7 and 8, it was confirmed that the example and intermediate compounds according to the present invention exhibit high inhibitory activity against cell growth of various RET mutations, including RET-KIF5B point mutations V804M, V804R, and G810R, and RET point mutation V804M.

In addition, as shown in Table 9, it was confirmed that the example and intermediate compounds according to the present invention exhibit high inhibitory activity against cell growth of the non-small cell lung cancer cell line A-549.

Therefore, it was confirmed that the compounds represented by Formula I or Formula IV according to the present invention not only exhibit high inhibitory activity against RET mutations, but also have high growth inhibitory activity against cells expressing RET mutations and non-small cell lung cancer cells, which are RET kinase-related diseases, and thus can be usefully used for the treatment of various cancers expressing RET and RET mutations.

From the above results, it can be seen that the quinoxaline derivative compounds according to the present invention can effectively inhibit RET and RET mutations, and thus can be usefully used as a pharmaceutical composition for the prevention or treatment of cancer.

### <Experimental Example 3> Evaluation of Enzyme Selectivity

To evaluate whether the compounds represented by Formula I or Formula IV according to the present invention selectively inhibit enzyme activity against RET and its mutations, the following experiment was performed. The results are shown in Table 10 and FIG. 1 below.

### 1) Experimental Method

The enzymatic inhibitory activity was measured in a similar manner to Experimental Example 1, and the residual activity (%) of the example compounds after inhibition against each kinase is shown in Table 10 and FIG. 1.

**[Table 10]**

| Example | % Enzyme Activity (relative to DMSO controls) at 1uM | | | | | |
|---|---|---|---|---|---|---|
| | RET | c-Kit | DDR1 | FLT3 | VEGFR3 | EPHB1 |
| 3 | 0.39±0.0 3 | 0.75±0.0 3 | 1.74±0.2 0 | 1.86±0.3 1 | 2.08±0.1 2 | 3.53±0.2 0 |

As shown in Table 10 and FIG. 1, the example compounds according to the present invention can selectively inhibit tyrosine kinase, and it can be seen that the inhibitory effect against receptor tyrosine kinase is particularly excellent. In particular, as the compounds exhibit the most potent inhibitory activity against RET kinase, they can be more selectively and effectively used for the treatment of cancers expressing RET and its mutations.

## Claims

1. A compound of Formula I or Formula IV below, or a pharmaceutically acceptable salt thereof: 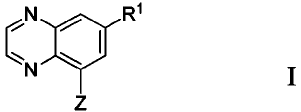 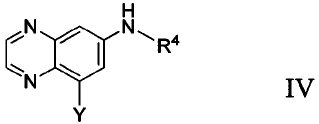 wherein, in Formulae I and IV,
Z is 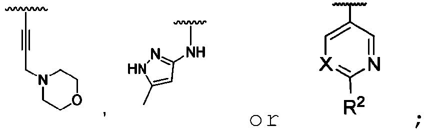
X is CH or N;
R¹ is NO₂ or -NR^{a}R^{b},
wherein each of R^{a} and R^{b} is independently H, C₁₋₁₀ alkyl, or 5-6 membered heteroarylcarbonyl;
R² is halogen or 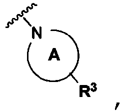
wherein ring A is a 4-6 membered heterocycloalkyl comprising at least one N atom of the point of attachment,
R³ is H, C₁₋₁₀ alkyl, C₁₋₁₀ alkylsulfonyl, C₁₋₃ alkyl substituted with unsubstituted or substituted phenyl, unsubstituted or substituted phenyloxy, unsubstituted or substituted phenyl-CONH-, 5-6 membered heteroaryl, or 6 membered heterocycloalkyl,
wherein the substituted phenyl is substituted with one or more halogens;
Y is halogen; and
R⁴ is C₁₋₁₀ alkyl or 5-6 membered heteroarylcarbonyl.

2. The compound or the pharmaceutically acceptable salt thereof according to claim 1, wherein:
R^{a} is H, and R^{b} is H, C₁₋₆ alkyl, or 5-6 membered heteroarylcarbonyl; or
each of R^{a} and R^{b} is independently C₁₋₆ alkyl.

3. The compound or the pharmaceutically acceptable salt thereof according to claim 1, wherein ring A is a 4-6 membered heterocycloalkyl optionally further comprising 1-2 or 1 additional N heteroatoms, or optionally further comprising 1 additional O heteroatom.

4. The compound or the pharmaceutically acceptable salt thereof according to claim 1, wherein R³ is H, C₁₋₆ alkyl, C₁₋₆ alkylsulfonyl, C₁₋₃ alkyl substituted with substituted phenyl, substituted phenyloxy, substituted phenyl-CONH-, 6 membered heteroaryl comprising 1 or 2 N heteroatoms, or morpholinyl,
wherein the substituted phenyl is substituted with 1 or 2 halogens.

5. The compound or the pharmaceutically acceptable salt thereof according to claim 1, wherein R⁴ is C₁₋₁₀ alkyl or 5-6 membered heteroarylcarbonyl.

6. The compound or the pharmaceutically acceptable salt thereof according to claim 1, wherein R⁴ is C₁₋₅ alkyl, furanylcarbonyl, or pyridylcarbonyl.

7. The compound or the pharmaceutically acceptable salt thereof according to claim 1, wherein:
X is CH or N;
R¹ is NO₂ or -NR^{a}R^{b},
wherein R^{a} is H or C₁₋₄ alkyl,
and R^{b} is H, C₁₋₄ alkyl, furanylcarbonyl, or pyridylcarbonyl;
ring A is a 4-6 membered heterocycloalkyl optionally further comprising 1 additional heteroatom selected from N and O together with the N atom at the point of attachment; and
R³ is H, C₁₋₃ alkyl, C₁₋₃ alkylsulfonyl, phenylmethyl substituted with 1 halogen, phenyloxy substituted with 1 halogen, phenyl-CONH- substituted with 1 halogen, pyridinyl, or morpholinyl.

8. The compound or the pharmaceutically acceptable salt thereof according to claim 1, wherein:
X is CH or N;
R¹ is amino, methylamino, ethylamino, propylamino, isopropylamino, butylamino, isobutylamino, furan-2-yl-CONH-, pyridin-2-yl-CONH-, diethylamino, or nitro; and
R² is 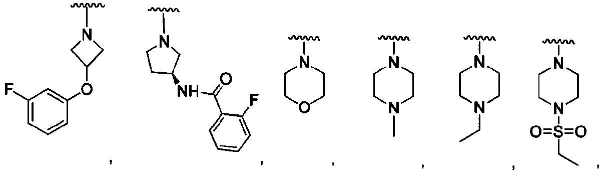 or F.

9. The compound, a stereoisomer thereof, or the pharmaceutically acceptable salt thereof according to claim 1, wherein the compound is selected from the group consisting of:
<1> 8-(6-(3-(3-fluorophenoxy)azetidin-1-yl)pyridin-3-yl)quinoxalin-6-amine;
<2> 8-(6-(3-(3-fluorophenoxy)azetidin-1-yl)pyridin-3-yl)-N-methylquinoxalin-6-amine;
<3> N-ethyl-8-(6-(3-(3-fluorophenoxy)azetidin-1-yl)pyridin-3-yl)quinoxalin-6-amine;
<4> 8-(6-(3-(3-fluorophenoxy)azetidin-1-yl)pyridin-3-yl)-N-propylquinoxalin-6-amine;
<5> 8-(6-(3-(3-fluorophenoxy)azetidin-1-yl)pyridin-3-yl)-N-isopropylquinoxalin-6-amine;
<6> N-butyl-8-(6-(3-(3-fluorophenoxy)azetidin-1-yl)pyridin-3-yl)quinoxalin-6-amine;
<7> 8-(6-(3-(3-fluorophenoxy)azetidin-1-yl)pyridin-3-yl)-N-isobutylquinoxalin-6-amine;
<8> N-(8-(6-(3-(3-fluorophenoxy)azetidin-1-yl)pyridin-3-yl)quinoxalin-6-yl)furan-2-carboxamide;
<9> N-(8-(6-(3-(3-fluorophenoxy)azetidin-1-yl)pyridin-3-yl)quinoxalin-6-yl)picolinamide;
<10> (S)-N-(1-(5-(7-aminoquinoxalin-5-yl)pyridin-2-yl)pyrrolidin-3-yl)-2-fluorobenzamide;
<11> (S)-2-fluoro-N-(1-(5-(7-(methylamino)quinoxalin-5-yl)pyridin-2-yl)pyrrolidin-3-yl)benzamide;
<12> (S)-N-(1-(5-(7-(ethylamino)quinoxalin-5-yl)pyridin-2-yl)pyrrolidin-3-yl)-2-fluorobenzamide;
<13> (S)-2-fluoro-N-(1-(5-(7-(propylamino)quinoxalin-5-yl)pyridin-2-yl)pyrrolidin-3-yl)benzamide;
<14> (S)-2-fluoro-N-(1-(5-(7-(isopropylamino)quinoxalin-5-yl)pyridin-2-yl)pyrrolidin-3-yl)benzamide;
<15> (S)-N-(1-(5-(7-(butylamino)quinoxalin-5-yl)pyridin-2-yl)pyrrolidin-3-yl)-2-fluorobenzamide;
<16> (S)-2-fluoro-N-(1-(5-(7-(isobutylamino)quinoxalin-5-yl)pyridin-2-yl)pyrrolidin-3-yl)benzamide;
<17> (S)-N-(8-(6-(3-(2-fluorobenzamido)pyrrolidin-1-yl)pyridin-3-yl)quinoxalin-6-yl)furan-2-carboxamide;
<18> (S)-N-(8-(6-(3-(2-fluorobenzamido)pyrrolidin-1-yl)pyridin-3-yl)quinoxalin-6-yl)picolinamide;
<19> 8-(6-morpholinopyridin-3-yl)quinoxalin-6-amine;
<20> 8-(6-(4-methylpiperazin-1-yl)pyridin-3-yl)quinoxalin-6-amine;
<21> 8-(6-(4-ethylpiperazin-1-yl)pyridin-3-yl)quinoxalin-6-amine;
<22> 8-(6-(4-(ethylsulfonyl)piperazin-1-yl)pyridin-3-yl)quinoxalin-6-amine;
<23> 8-(6-(4-(pyridin-2-yl)piperazin-1-yl)pyridin-3-yl)quinoxalin-6-amine;
<24> 8-(6-(4-(4-bromobenzyl)piperazin-1-yl)pyridin-3-yl)quinoxalin-6-amine;
<25> 8-(6-(4-morpholinopiperidin-1-yl)pyridin-3-yl)quinoxalin-6-amine;
<26> 8-(6-(4-(4-bromobenzyl)piperazin-1-yl)pyridin-3-yl)-N-ethylquinoxalin-6-amine;
<27> 5-(6-(3-(3-fluorophenoxy)azetidin-1-yl)pyridin-3-yl)-7-nitroquinoxaline;
<28> N,N-diethyl-8-(6-(3-(3-fluorophenoxy)azetidin-1-yl)pyridin-3-yl)quinoxalin-6-amine;
<29> 8-(2-(3-(3-fluorophenoxy)azetidin-1-yl)pyrimidin-5-yl)quinoxalin-6-amine;
<30> (S)-N-(1-(5-(7-aminoquinoxalin-5-yl)pyrimidin-2-yl)pyrrolidin-3-yl)-2-fluorobenzamide;
<31> N-ethyl-8-(3-morpholinoprop-1-yn-1-yl)quinoxalin-6-amine; and
<32> N7-ethyl-N5-(5-methyl-1H-pyrazol-3-yl)quinoxaline-5,7-diamine.

10. The compound, a stereoisomer thereof, or the pharmaceutically acceptable salt thereof according to claim 1, wherein the compound is selected from the group consisting of:
<1> 8-bromo-N-butylquinoxalin-6-amine;
<2> 8-bromo-N-isobutylquinoxalin-6-amine;
<3> 8-(6-fluoropyridin-3-yl)quinoxalin-6-amine;
<4> 8-(6-fluoropyridin-3-yl)-N-methylquinoxalin-6-amine;
<5> N-ethyl-8-(6-fluoropyridin-3-yl)quinoxalin-6-amine;
<6> 8-(6-fluoropyridin-3-yl)-N-propylquinoxalin-6-amine;
<7> 8-(6-fluoropyridin-3-yl)-N-isopropylquinoxalin-6-amine;
<8> N-butyl-8-(6-fluoropyridin-3-yl)quinoxalin-6-amine;
<9> 8-(6-fluoropyridin-3-yl)-N-isobutylquinoxalin-6-amine;
<10> N-(8-bromoquinoxalin-6-yl)furan-2-carboxamide;
<11> N-(8-bromoquinoxalin-6-yl)picolinamide;
<12> N-(8-(6-fluoropyridin-3-yl)quinoxalin-6-yl)furan-2-carboxamide;
<13> N-(8-(6-fluoropyridin-3-yl)quinoxalin-6-yl)picolinamide;
<14> 5-(6-fluoropyridin-3-yl)-7-nitroquinoxaline;
<15> 8-(2-chloropyrimidin-5-yl)quinoxalin-6-amine;
<16> 8-bromo-N-ethylquinoxalin-6-amine;
<17> 8-bromo-N-propylquinoxalin-6-amine; and
<18> 8-bromo-N-isopropylquinoxalin-6-amine.

11. A method of preparing a compound of Formula IA according to claim 1, comprising reacting a compound of Formula II with a compound of Formula III as shown in Reaction Scheme I below: 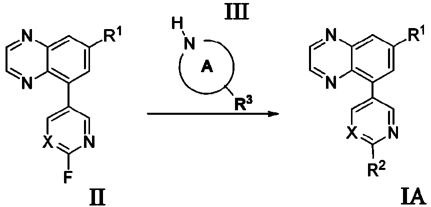 wherein X, R¹, R², ring A, and R³ are as defined in Formula I of claim 1.

12. A pharmaceutical composition for the prevention or treatment of cancer, comprising the compound or the pharmaceutically acceptable salt thereof according to claim 1 as an active ingredient.

13. The pharmaceutical composition according to claim 12, wherein the compound selectively inhibits tyrosine kinase.

14. The pharmaceutical composition according to claim 13, wherein the kinase is a receptor tyrosine kinase.

15. The pharmaceutical composition according to claim 13, wherein the kinase comprises one or more selected from the group consisting of RET, c-Kit (Tyrosine-protein kinase KIT, CD117), DDR1 (Discoidin domain receptor family, member 1), FLT3 (fms related receptor tyrosine kinase 3), VEGFR3 (Vascular endothelial growth factor receptor 3), and EPHB1 (Ephrin type-B receptor 1).

16. A pharmaceutical composition for the prevention or treatment of a disease associated with RET (Rearranged During Transfection) or a mutation thereof, comprising the compound or the pharmaceutically acceptable salt thereof according to claim 1 as an active ingredient.

17. The pharmaceutical composition according to claim 16, wherein the RET mutation comprises one or more selected from the group consisting of a point mutation, a fusion mutation, and a multiple mutation.

18. The pharmaceutical composition according to claim 17, wherein the point mutation comprises one or more selected from the group consisting of RET(G810R), RET(V804M), and RET(G810S/M918T).

19. The pharmaceutical composition according to claim 17, wherein the fusion mutation comprises one or more selected from the group consisting of KIF5B-RET, RET-CCDC6, and RET-NCOA4.

20. The pharmaceutical composition according to claim 17, wherein the multiple mutation comprises one or more selected from the group consisting of KIF5B-RET, KIF5B-RET(V804M), KIF5B-RET(G810R), RET(V804M)-KIF5B, and RET(G810S/M918T).

21. The pharmaceutical composition according to claim 12, wherein the cancer is one or more selected from the group consisting of pseudomyxoma, intrahepatic cholangiocarcinoma, hepatoblastoma, hepatocellular carcinoma, thyroid cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell tumor, male breast cancer, brain cancer, pituitary adenoma, multiple myeloma, gallbladder cancer, cholangiocarcinoma, colorectal cancer, chronic myeloid leukemia, chronic lymphocytic leukemia, retinoblastoma, choroidal melanoma, ampulla of vater cancer, bladder cancer, peritoneal cancer, parathyroid cancer, adrenal cancer, sinonasal cancer, tongue cancer, astrocytoma, small intestine cancer, meningioma, esophageal cancer, glioma, renal pelvis cancer, renal cancer, cardiac cancer, duodenal cancer, malignant soft tissue cancer, malignant bone cancer, malignant lymphoma, malignant mesothelioma, malignant melanoma, ocular cancer, vulvar cancer, ureteral cancer, urethral cancer, cancer of unknown primary, gastric lymphoma, gastric cancer, gastric carcinoid tumor, gastrointestinal stromal tumor, Wilms tumor, breast cancer, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, vaginal cancer, spinal cord cancer, acoustic neuroma, pancreatic cancer, salivary gland cancer, Kaposi's sarcoma, Paget's disease, tonsillar cancer, squamous cell carcinoma, lung cancer, skin cancer, anal cancer, rhabdomyosarcoma, laryngeal cancer, pleural cancer, and thymic cancer.

22. A dietary supplement composition for the prevention or amelioration of cancer, comprising the compound or the pharmaceutically acceptable salt thereof according to claim 1 as an active ingredient.

23. A method of treating cancer, comprising administering a therapeutically effective amount of the compound or the pharmaceutically acceptable salt thereof according to claim 1 to a subject in need thereof.

24. A use of the compound or the pharmaceutically acceptable salt thereof according to claim 1 for the preparation of a medicament for the prevention or treatment of cancer.
